(19) Europäisches Patentamt
European Patent Office
Office européen des brevets

(11) EP 4 056 681 A1

(12) EUROPEAN PATENT APPLICATION

(43) Date of publication:
14.09.2022 Bulletin 2022/37

(21) Application number: 21162331.9

(22) Date of filing: 12.03.2021

(51) International Patent Classification (IPC):
*C12N 5/077* (2010.01) *G01N 21/64* (2006.01)
*G01N 33/487* (2006.01)

(52) Cooperative Patent Classification (CPC):
**G01N 21/6408; A01K 67/0336; C07K 14/00; G01N 33/48728;** G01N 2021/6439

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Johann Wolfgang Goethe-Universität Frankfurt**
**60323 Frankfurt am Main (DE)**

(72) Inventors:
- **Gottschalk, Alexander**
**61118 Bad Vilbel (DE)**
- **Bergs, Amelie**
**60439 Frankfurt am Main (DE)**

(74) Representative: **Grahn, Sibylla Maria**
**Boehmert & Boehmert**
**Anwaltspartnerschaft mbB**
**Pettenkoferstrasse 22**
**80336 München (DE)**

# (54) AN OPTOGENETIC VOLTAGE CLAMP (OVC) AND USES THEREOF

(57) The present invention relates to using a genetically encoded voltage indicator (GEVI) protein and counteracting optogenetic protein(s) for depolarization and hyperpolarization as an optic voltage clamp with feedback control in a cell. The present invention further relates to cells co-expressing a GEVI protein, and counteracting optogenetic protein(s) for depolarization and hyperpolarization and their uses. The present invention further relates to a method for controlling the membrane voltage of a target cell and to a method for determining the functionality of or modulating ion channels and/or for determining influences on ion channels.

EP 4 056 681 A1

SEQUENCE LISTING

<110>  Johann Wolfgang Goethe-Universität Frankfurt am Main

<120>  An optogenetic voltage clamp (OVC) and uses thereof

<130>  U31051EP

<160>  14

<170>  PatentIn version 3.5

<210>  1
<211>  38
<212>  DNA
<213>  Artificial Sequence

<220>
<223>  Primer

<400>  1
atttcagga ggacccttgg atggcatcac aggtcgtc    38

<210>  2
<211>  38
<212>  DNA
<213>  Artificial Sequence

<220>
<223>  Primer

<400>  2
ataccatggt accgtcgacg tcacactgtg tcctcgtc    38

<210>  3
<211>  35
<212>  DNA
<213>  Artificial Sequence

<220>
<223>  Primer

<400>  3
actagatcca tctagagatg gcatcacagg tcgtc    35

<210>  4
<211>  33
<212>  DNA
<213>  Artificial Sequence

<220>
<223>  Primer

<400>  4
ttggccaatc ccgggcactg tgtcctcgtc ctc    33

<210>  5
<211>  38
<212>  DNA

<213> Artificial Sequence

<220>
<223> Primer

<400> 5
ttacagcacc ggtggattgg atggtaagta tcgctctg 38

<210> 6
<211> 43
<212> DNA
<213> Artificial Sequence

<220>
<223> Primer

<400> 6
ttctacgaat gctcctaggc ctatttgtat agttcatcca tgc 43

<210> 7
<211> 28
<212> DNA
<213> Artificial Sequence

<220>
<223> Primer

<400> 7
caccgagtga gaagagcagg atcaccag 28

<210> 8
<211> 42
<212> DNA
<213> Artificial Sequence

<220>
<223> Primer

<400> 8
tgcagagcga tacttaccat ccccgagggt taaaatgaaa ag 42

<210> 9
<211> 21
<212> DNA
<213> Artificial Sequence

<220>
<223> Primer

<400> 9
atggtaagta tcgctctgca g 21

<210> 10
<211> 33
<212> DNA
<213> Artificial Sequence

<220>

<223> Primer

<400> 10
cctgctcttc tcactcggtg tcgcccagaa tag 33

<210> 11
<211> 38
<212> DNA
<213> Artificial Sequence

<220>
<223> Primer

<400> 11
ggacgaggac acagtgtgaa aagagcagga tcaccagc 38

<210> 12
<211> 42
<212> DNA
<213> Artificial Sequence

<220>
<223> Primer

<400> 12
agacgacctg tgatgccatg ccccgagggt taaaatgaaa ag 42

<210> 13
<211> 38
<212> DNA
<213> Artificial Sequence

<220>
<223> Primer

<400> 13
acatttattt cattacagca atggcatcac aggtcgtc 38

<210> 14
<211> 38
<212> DNA
<213> Artificial Sequence

<220>
<223> Primer

<400> 14
agcgaccggc gctcagttgg tcacactgtg tcctcgtc 38

## Description

The present invention relates to using a genetically encoded voltage indicator (GEVI) protein and counteracting optogenetic protein(s) for depolarization and hyperpolarization as an optic voltage clamp with feedback control in a cell. The present invention further relates to cells co-expressing a GEVI protein, and counteracting optogenetic protein(s) for depolarization and hyperpolarization and their uses. The present invention further relates to a method for controlling the membrane voltage of a target and to a method for determining the functionality of or modulating ion channels and/or for determining influences on ion channels.

## BACKGROUND OF THE INVENTION

Identifying connections between distinct neurons within neural circuits and their contribution to the behaviours they drive is a central issue in neuroscience research.

Nowadays, a broad range of approaches to control or observe excitable cell function are used. Traditional patch-clamp electrophysiology provides superior temporal accuracy and sensitivity (Francis *et al.,* 2003), yet it is limited by its highly invasive character. Calcium imaging can be applied in intact living organisms and thus enables to integrate behavioural aspects (Akerboom *et al.,* 2013; Chen *et al.,* 2013; Lin & Schnitzer, 2016). However, this technique suffers from comparatively low temporal resolution, thus fails to resolve voltage transients or highfrequency action potential (APs) for most neuron types. Furthermore, it offers no direct measure of membrane voltage, thus making it unsuitable to reveal synaptic inhibition, since typically the $Ca^{2+}$ concentration does not fall below basal cytosolic levels. This gap can be bridged by genetically encoded (fluorescent) voltage indicators (GEVIs), particularly the rhodopsin-based GEVIs (Kralj *et al.,* 2011; Gong *et al.,* 2013; Gong *et al.,* 2014; Fan *et al.,* 2020; Hochbaum *et al.,* 2014; Piatkevich *et al.,* 2018). The fluorescence of retinal, embedded in this kind of proteins, albeit dim and thus requiring intense excitation, reliably monitors membrane voltage dynamics at sub-millisecond time scales. Furthermore, neural imaging tools can be multiplexed with optogenetic actuators, which employ light to selectively stimulate activity of excitable cells in living tissue at high temporal and spatial precision, providing actuation of neural function. However, expressing single optogenetic actuators aside with imaging tools allows only to perturb and passively observe, but not to control the activity within a neural circuit (Hochbaum *et al.,* 2014; Fan *et al.,* 2020). This unidirectional approach was implemented with the so-called "(i)-Optopatch" method, consisting of the voltage indicator QuasAr and the blue-light-shifted Channelrhodopsin (ChR) CheRiff (Hochbaum *et al.,* 2014; Fan *et al.,* 2020). An advanced bidirectional approach, comprising ChR2 and NpHR as optogenetic actuation pair and feedback control - "optoclamp" - uses extracellular microelectrode arrays instead of GEVIs, thus being limited to the observation and clamping of neuronal populations' firing levels instead of single neurons and direct voltage readout, as well as a rather low temporal resolution in the range of seconds to dozens of seconds (Newman *et al.,* 2015).

Identifying connections between distinct neurons within neural circuits and their contribution to the behaviours they drive is a central issue in neuroscience research. To shed light on these relations, a non-invasive method to record and at the same time regulate neural activity is desirable.

It is an objective of the present invention to provide means and methods for controlling membrane voltage in cells and tissues.

## SUMMARY OF THE INVENTION

According to the present invention this object is solved by using a genetically encoded voltage indicator (GEVI) protein and counteracting optogenetic protein(s) for depolarization and hyperpolarization as an optic voltage clamp in a cell.

According to the present invention this object is solved by using a genetically encoded voltage indicator (GEVI) protein and counteracting optogenetic protein(s) for depolarization and hyperpolarization for optical control of the membrane potential in a cell.

According to the present invention this object is solved by a cell co-expressing a genetically encoded voltage indicator (GEVI) protein, and counteracting optogenetic protein(s) for depolarization and hyperpolarization.

According to the present invention this object is solved by using the cell according to the present invention for

optical control of membrane potential,
studying muscular ensembles or neuronal networks,
studying animal models,
studying or providing models of neurodegenerative diseases,
studying or providing models of cardiac diseases,
functional characterization of ion channels,

expression analysis screening of ion channels, and/or
screening for drugs that affect ion channels.

According to the present invention this object is solved by a method for controlling the membrane voltage of a target cell,
the method comprising:

(1) incorporating into a target cell a voltage indicator, and counteracting optogenetic protein(s) for depolarization and hyperpolarization;
or providing a cell according to the present invention as target cell,
(2) exciting the fluorescence of the voltage indicator with a first light source,
(3) recording the intensity of fluorescence with a camera in real time and determining the relative change of fluorescence ($\Delta F/F_0$),
(4) at the same time as (2) and (3), irradiating the counteracting optogenetic protein(s) for depolarization and hyperpolarization with a second light source,
comprising the adjustment of a feedback wavelength of said second light source depending on (i) the difference between intended membrane voltage and present membrane voltage, reflected by relative changes in fluorescence ($\Delta F/F_0$),and (ii) the absorption maxima of the optogenetic protein(s);
wherein said feedback wavelength is adjusted

(a) towards the absorption maximum of the depolarizing tool, if the present membrane voltage is greater than a target membrane voltage, whereas the membrane voltage is indicated by the relative change in fluorescence ($\Delta F/F_0$),and
(b) towards the absorption maximum of the hyperpolarizing tool, if the present membrane voltage is greater than a target membrane voltage, whereas the membrane voltage is indicated by the relative change in fluorescence ($\Delta F/F_0$),

to clamp the membrane potential at the target voltage, reflected by the target $\Delta F/F_0$ value, which results in an optical closed-loop controlling of the membrane potential of the target cell.

According to the present invention this object is solved a method for determining the functionality of and/or for modulating ion channels and/or for determining influences on ion channels,
the method comprising:

(1) providing a cell comprising the ion channel to be determined for functionality or modulated, and incorporating into said cell a voltage indicator, and counteracting optogenetic protein(s) for depolarization and hyperpolarization;
or providing a cell according to the present invention as target cell and introducing the ion channel to be determined for functionality or modulated,
comprising using ion channel variant(s) comprising mutation(s) and/or adding compound(s) to be tested for an effect on the ion channel or its variant(s),
(2) exciting the fluorescence of the voltage indicator with a first light source,
(3) recording the intensity of fluorescence with a camera in real time and determining the relative change of fluorescence ($\Delta F/F_0$),
(4) at the same time as (2) and (3), irradiating the counteracting optogenetic protein(s) for depolarization and hyperpolarization with a second light source,
comprising the adjustment of a feedback wavelength of said second light source depending on (i) the difference between intended membrane voltage and present membrane voltage, reflected by relative changes in fluorescence ($\Delta F/F_0$),and (ii) the absorption maxima of the optogenetic protein(s);
wherein said feedback wavelength is adjusted

(a) towards the absorption maximum of the depolarizing tool, if the present membrane voltage is smaller than a target membrane voltage, whereas the membrane voltage is indicated by the relative change in fluorescence ($\Delta F/F_0$),and
(b) towards the absorption maximum of the hyperpolarizing tool, if the present membrane voltage is greater than a target membrane voltage, whereas the membrane voltage is indicated by the relative change in fluorescence ($\Delta F/F_0$),

to clamp the membrane potential at the intended membrane voltage, reflected by the target $\Delta F/F_0$ value,

which results in an optical closed-loop, and

(5) determining the feedback light parameters of step (4) and comparing them with the feedback light parameters determined for a control, wherein a difference between said feedback light parameters of step (4) and the control indicates that the mutation(s) of the ion channel and/or the added compound(s) have an effect on the ion channel, wherein the feedback light parameters are selected from the feedback wavelength(s), the course of the feedback wavelength(s) and/or the light intensity, and wherein the control is the wildtype ion channel and/or no compound(s) are added.

DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 20" should be interpreted to include not only the explicitly recited values of 1 to 20, but also include individual values and sub-ranges within the indicated range. Thus, included in this numerical range are individual values such as 1, 2, 3, 4, 5 .... 17, 18, 19, 20 and sub-ranges such as from 2 to 10, 8 to 15, etc. This same principle applies to ranges reciting only one numerical value, such as "higher than 150 mg per day". Furthermore, such an interpretation should apply regardless of the breadth of the range or the characteristics being described.

*An optogenetic voltage clamp (OVC)*

As outlined above, the present invention provides the use of a genetically encoded voltage indicator (GEVI) protein and counteracting optogenetic protein(s) for depolarization and hyperpolarization as an optic voltage clamp in a cell.

As outlined above, the present invention provides the use of genetically encoded voltage indicator (GEVI) protein and counteracting optogenetic protein(s) for depolarization and hyperpolarization for optical control of the membrane potential in a cell.

*- Cells*

The cell in which the membrane potential is controlled with the optogenetic voltage clamp (OVC) according to the present invention can be any cell, in particular any cell in which a membrane potential is intrinsically determined or dynamically altered. It is a cell in which membrane potential is subject to dynamic changes or where changes in membrane potential affect membrane transport processes, for example through secondary active transporters.

In a preferred embodiment, the cell is an electrically excitable cell, such as a muscle cell, a neuron, a cardiomyocyte (such as iPSC-derived cardiomyocyte) or a glial cell.

In one embodiment, the cell is a cell of cultured cell lines known to the skilled artisan, such as HeLa or HEK cells.

In one embodiment, the cell is a cell of cultured cell lines used in the art for expressing ion channels, such as stable cell lines.

*- Components of the OVC*

The present invention provides an optical voltage clamp, which comprises a voltage indicator and counteracting optogenetic tools.

Preferably, the optic voltage clamp is an optogenetic voltage clamp (OVC).

In an optogenetic voltage clamp (OVC) both the voltage indicator and the counteracting optogenetic tools are proteins.

The *voltage indicator* is preferably a genetically encoded voltage indicator (GEVI) protein.

The terms "genetically encoded voltage indicator protein" and "genetically encoded voltage indicator" are used interchangeably in this application.

A genetically encoded voltage indicator (or GEVI) is a protein that can sense membrane potential in a cell and relate the change in voltage to a form of output, often fluorescent level.

It is an optogenetic recording tool that enables exporting electrophysiological signals from (cultured) cells, live animals, tissues, such as (human) brain.

GEVIs are known in the art. A GEVI can have differing configuration designs in order to realize voltage sensing function. An essential feature of GEVI structure is that it must situate on the cell membrane. Conceptually, the structure of a GEVI should permit the function of sensing the voltage difference and reporting it by change in fluorescence. Usually, the voltage-sensing domain (VSD) of a GEVI spans across the membrane, and is connected to the fluorescent protein(s) (FP). By structure, GEVIs can be classified into four categories: (1) GEVIs contain a fluorescent protein FRET pair, e.g. VSFP1, (2) Single opsin GEVIs, e.g. Arch, QuasAr, (3) Opsin-FP FRET pair GEVIs, e.g. MacQ-mCitrine, (4) single FP with special types of voltage sensing domains, e.g. ASAP1. A majority of GEVIs are based on the *Ciona intestinalis* voltage sensitive phosphatase (Ci-VSP or Ci-VSD (domain)). Some GEVIs might have similar components, but with different positioning of them. For example, ASAP1 and ArcLight both use a VSD and one FP, but the FP of ASAP1 is on the outside of the cell whereas that of ArcLight is on the inside, and the two FPs of VSFP-Butterfly are separated by the VSD, while the two FPs of Mermaid are relatively close to each other.

Preferably, the GEVI protein is

- an opsin-based GEVI protein
  such as rhodopsin-based,

    e.g. Arch, QuasAr, QuasAr2, paQuasAr3, Archon, SomArchon, or eFRET-based or FP-retinal FRET,
    e.g. Ace-mNeon, VARNAM, macQ-mCitrine, QuasAr2-mOrange2, paQuasAr3,

  or
- a voltage-sensing domain (VSD)-based GEVI protein

    such as a FP-FP FRET,
    e.g. Mermaid, VSFP Butterflies, or
    a monochromatic FP,
    e.g. FlicR, ArcLight, ASAP.

The *counteracting optogenetic tools* are counteracting optogenetic protein(s) for depolarization and hyperpolarization.

In one embodiment, the counteracting optogenetic proteins for depolarization and hyperpolarization are (i) a pair of optogenetic proteins, wherein one is for depolarization and the other is for hyperpolarization.

In one embodiment, the counteracting optogenetic proteins for depolarization and hyperpolarization are (ii) a bidirectional optogentic protein.

Such counteracting optogenetic protein(s) are known in the art.

Preferably, the depolarizing optogenetic proteins are:

- channelrhodopsins (ChRs), such as ChR1, ChR2, VChR1, ChRmine, or
- ChR2 variants or chimeras, such as Chrimson, ReaChR, CheRiff, ChETA, bReaChEs.

Preferably, the hyperpolarizing optogenetic proteins are:

- halorhodopsins, such as eNpHR2.0 and eNpHR3.0,
- Archaerhodopsins,
- fungal opsins, such as Mac,
- bacteriorhodopsins, such as eBR,
- anion-conducting channelrhodopsins (ACRs), such as iC1C2, *Gt*ACR1, *Gt*ACR2, Phobos, Aurora, (i-)ChloCs.

Examples for pairs of optogenetic proteins for depolarization and hyperpolarization are

- NpHR and ChR2,
- ACRs, such as ACR1, and channelrhodopsin (ChR)
- GtACR2 and Chrimson.

Bidirectional optogenetic proteins can be fusion proteins of any of the above described depolarizing and hyperpolarizing optogenetic proteins, such as depolarizing and hyperpolarizing rhodopsins, whose action spectral peaks are sufficiently separated, preferably by about 75-100 nm, more preferably about 100-150 nm or more.

Preferably, a bidirectional optogentic protein is:

- a fusion protein of blue-light-sensitive anion-conducting channelrhodopsin GtACR2 fused to the red-light-sensitive cation-conducting channelrhodopsin Chrimson, GtACR2 fused to Chrimson (BiPOLES),
- GtACR2 fused to bReaChEs,
- GtACR2 fused to ChRmine.

In one embodiment, the use of the present invention comprises studying muscular ensembles or neuronal networks, such as how they control or mediate behavior.

In one embodiment, the use of the present invention comprises studying animal models, which are preferably transparent.

In one embodiment, the use of the present invention comprises studying or providing models of neurodegenerative diseases.

In one embodiment, the use of the present invention comprises studying or providing models of cardiac diseases.

In one embodiment, the use of the present invention comprises functional characterization of ion channels.

In one embodiment, the use of the present invention comprises expression analysis screening of ion channels.

In one embodiment, the use of the present invention comprises screening for drugs that affect ion channels.

The ion channels can be ryanodine receptor, sodium channels, calcium channels, potassium channels (such as HERG K+ channel), and ligand-gated ion channels.

Thereby, also intracellular ion channels, such as ryanodine receptor, can be screened.

Preferably, the screening of ion channels or the drug screening is carried out as a high-throughput screening.

In a preferred embodiment, cells of a patient with a neurodegenerative disease, epilepsy, a cardiac disease (such as heart rhythm dysfunction), a renal disease, a muscular malfunction based on ion channel mutations, a disease based on aberrant mitochondrial membrane potential and/or a disease based on aberrant organellar membrane potential are used.

*Cells expressing the OVC and their uses*

As outlined above, the present invention provides a cell co-expressing

a genetically encoded voltage indicator (GEVI) protein, and
counteracting optogenetic protein(s) for depolarization and hyperpolarization.

The cell according to the present invention can be any cell, in particular any cell in which a membrane potential is intrinsically determined or dynamically altered. It is a cell in which membrane potential is subject to dynamic changes or where changes in membrane potential affect membrane transport processes, for example through secondary active transporters.

In a preferred embodiment, the cell is an electrically excitable cell, such as a muscle cell, a neuron, a cardiomyocyte (such as iPSC-derived cardiomyocyte) or a glial cell.

In one embodiment, the cell is a cell of cultured cells lines known to the skilled artisan, such as HeLa or HEK cells.

In one embodiment, the cell is a cell of cultured cells line used in the art for expressing ion channels, such as stable cell lines.

The GEVI protein is preferably as defined above.

The counteracting optogenetic protein(s) for depolarization and hyperpolarization are preferably as defined above.

As outlined above, the present invention provides the use of the cell according to the present invention for optical control of membrane potential.

As outlined above, the present invention provides the use of the cell according to the present invention for studying muscular ensembles or neuronal networks, such as how they control or mediate behavior.

As outlined above, the present invention provides the use of the cell according to the present invention for studying animal models, which are preferably transparent.

As outlined above, the present invention provides the use of the cell according to the present invention for studying or providing models of neurodegenerative diseases.

As outlined above, the present invention provides the use of the cell according to the present invention for studying or providing models of cardiac diseases.

As outlined above, the present invention provides the use of the cell according to the present invention for functional characterization of ion channels.

As outlined above, the present invention provides the use of the cell according to the present invention for expression analysis screening of ion channels.

As outlined above, the present invention provides the use of the cell according to the present invention for screening for drugs that affect ion channels.

Preferably, the ion channels can be ryanodine receptor, sodium channels, calcium channels, potassium channels (such as HERG K+ channel), and ligand-gated ion channels.
Thereby, also intracellular ion channels, such as ryanodine receptor, can be screened.

Preferably, the screening of ion channels or the drug screening is carried out as a high-throughput screening.

In a preferred embodiment, cells of a patient with a neurodegenerative disease, epilepsy, a cardiac disease (such as heart rhythm dysfunction), a renal disease, a muscular malfunction based on ion channel mutations, a disease based on aberrant mitochondrial membrane potential and/or a disease based on aberrant organellar membrane potential are used.

*Closed-loop methods with the OVC*

As outlined above, the present invention provides a *method for controlling the membrane voltage of a target cell.*

Said method comprises the steps of:

(1) incorporating into a target cell a voltage indicator, and counteracting optogenetic protein(s) for depolarization and hyperpolarization;
or providing a cell according to the present invention,

(2) exciting the fluorescence of the voltage indicator with a first light source,

(3) recording the intensity of fluorescence with a camera in real time and determining the relative change of fluorescence ($\Delta F/F_0$),

(4) at the same time as (2) and (3), irradiating the counteracting optogenetic protein(s) for depolarization and hyperpolarization with a second light source,
comprising the adjustment of a feedback wavelength of said second light source depending on (i) the difference between intended membrane voltage and present membrane voltage, reflected by relative changes in fluorescence ($\Delta F/F_0$),and (ii) the absorption maxima of the optogenetic protein(s);
wherein said feedback wavelength is adjusted

(a) towards the absorption maximum of the depolarizing tool, if the present membrane voltage is smaller than a target membrane voltage, whereas the membrane voltage is indicated by the relative change in fluorescence ($\Delta F/F_0$)and
(b) towards the absorption maximum of the hyperpolarizing tool, if the present membrane voltage is greater than a target membrane voltage, whereas the membrane voltage is indicated by the relative change in fluorescence ($\Delta F/F_0$),

to clamp the membrane potential at the target voltage, reflected by the target $\Delta F/F_0$ value,
which results in an optical closed-loop controlling of the membrane potential of the target cell.

The method of the present invention preferably further comprises a calibration and bleaching correction.
Said calibration and bleaching correction preferably comprises measuring the exponential decrease in fluorescence of the voltage indicator, such as a GEVI protein, and real-time correcting each incoming $\Delta F/F_0$ value accordingly.

The method of the present invention preferably further comprises a compensatory wavelength to counteract optical crosstalk.
Said which compensatory wavelength is preferably selected in case of (full) pre-activation of (one of) the tools and enables both the calculation of the starting fluorescence (Fo) as well as the bidirectional control / clamping of the membrane potential.

*Step (1) target cells with OVC*

The target cell provided in step (1) can be any cell, in particular any cell in which a membrane potential is intrinsically determined or dynamically altered. It is a cell in which membrane potential is subject to dynamic changes or where changes in membrane potential affect membrane transport processes, for example through secondary active transporters.

In a preferred embodiment, the target cell is an electrically excitable cell, such as a muscle cell, a neuron, a cardiomyocyte (such as iPSC-derived cardiomyocyte) or a glial cell.

The voltage indicator is a genetically encoded voltage indicator (GEVI) protein or a voltage sensitive dye.

Preferably, the GEVI protein is as defined herein.

Voltage sensitive dyes are known in the art. Voltage-sensitive dyes, also known as potentiometric dyes, are dyes which change their spectral properties in response to voltage changes. Examples are substituted aminonaphthylethenyl-pyridinium (ANEP) dyes, such as di-4-ANEPPS, di-8-ANEPPS, and RH237.

Into the target cell, a genetically encoded voltage indicator (GEVI) protein and counteracting optogenetic protein(s) for depolarization and hyperpolarization are introduced.
The target cell then co-expresses said GEVI and said counteracting optogenetic protein(s).

In a preferred embodiment, the target cell provided is a cell according to the present invention.

Preferably the counteracting optogenetic protein(s) for depolarization and hyperpolarization are as defined herein.

*Steps (2) and (3) -fluorescence of voltage indicator*

In step (2), the fluorescence of the voltage indicator, preferably a GEVI protein, is excited with a first light source.

The first light source is preferably a laser, an LED or an HBO lamp.

In step (3), the fluorescence emitted from the voltage indicator, preferably a GEVI protein, is recorded with a camera in real time.
From the intensity of fluorescence the relative change of fluorescence is determined ($\Delta F/F_0$).

The fluorescence recorded in step (3) is in linear dependence of the membrane voltage, i.e. depolarization results in increase of fluorescence, hyperpolarization results in decrease of fluorescence.
In one embodiment, in step (3) the camera records gray values and a software translates them into relative changes of fluorescence.

*Step (4) - controlling the membrane voltage through the counteracting optogenetic protein(s)*

Step (4) is carried out at the same time as steps (2) and (3), in parallel.

In step (4), the counteracting optogenetic protein(s) for depolarization and hyperpolarization are irradiated with a second light source.

The second light source is preferably a tunable light source, which can access a broad spectrum of wavelengths, but produces a narrow range of any of those wavelengths at a given time, such as a monochromator, a tunable laser.

Step (4) comprises the adjustment of a feedback wavelength of said second light source depending on (i) the difference between intended membrane voltage and present membrane voltage, reflected by relative changes in fluorescence ($\Delta F/F_0$),and (ii) the absorption maxima of the optogenetic protein(s).

Said feedback wavelength is adjusted

(a) towards the absorption maximum of the depolarizing tool, if the present membrane voltage is smaller than a target membrane voltage, whereas the membrane voltage is indicated by the relative change in fluorescence ($\Delta F/F_0$), and
(b) towards the absorption maximum of the hyperpolarizing tool, if the present membrane voltage is greater than a target membrane voltage, whereas the membrane voltage is indicated by the relative change in fluorescence ($\Delta F/F_0$),

to clamp the membrane potential at the target voltage, reflected by the target $\Delta F/F_0$ value,
which results in an optical closed-loop controlling of the membrane potential of the target cell.

As discussed above, the method preferably furthermore comprises one or more of the following:

(i) selecting a tolerance range for the difference between intended membrane voltage and present membrane voltage, reflected by relative changes in fluorescence ($\Delta F/F_0$),
preferably 0.1 to 10%, more preferably 0.2 to 5%, such as 1%;

(ii) weighting of the feedback light depending on the size of deviation between intended membrane voltage and present membrane voltage, reflected by relative changes in fluorescence ($\Delta F/F_0$),

(iii) adapting the wavelength of the second light source in real time in increments, which depend on the size of deviation between intended membrane voltage and present membrane voltage, reflected by relative changes in fluorescence ($\Delta F/F_0$),as soon as the present $\Delta F/F_0$ value violates a selected tolerance range,

(iv) preselecting the target membrane voltage, as indicated by the relative change in fluorescence ($\Delta F/F_0$), or changing it in real time.

*Methods for screening ion channels*

As outlined above, the present invention provides a *method for determining the functionality of ion channels or modulating ion channels and/or for determining influences on ion channels.*

Said method comprises the steps of:

(1) providing a cell comprising the ion channel to be determined for functionality or modulated, and incorporating into said cell a voltage indicator, and counteracting optogenetic protein(s) for depolarization and hyperpolarization; or providing a cell according to the present invention as target cell and introducing the ion channel to be determined or modulated,
comprising using ion channel variant(s) comprising mutation(s) and/or adding compound(s) to be tested for an effect on the ion channel or its variant(s),

(2) exciting the fluorescence of the voltage indicator with a first light source,

(3) recording the intensity of fluorescence with a camera in real time and determining the relative change of fluorescence ($\Delta F/F_0$),

(4) at the same time as (2) and (3), comprising the adjustment of a feedback wavelength of said second light source depending on (i) the difference between intended membrane voltage and present membrane voltage, reflected by relative changes in fluorescence ($\Delta F/F_0$),and (ii) the absorption maxima of the optogenetic protein(s);
wherein said feedback wavelength is adjusted

(a) towards the absorption maximum of the depolarizing tool, if the present membrane voltage is smaller than a target membrane voltage, whereas the membrane voltage is indicated by the relative change in fluorescence ($\Delta F/F_0$),and
(b) towards the absorption maximum of the hyperpolarizing tool, if the present membrane voltage is greater than a target membrane voltage, whereas the membrane voltage is indicated by the relative change in fluorescence ($\Delta F/F_0$),

to clamp the membrane potential at the intended membrane voltage, reflected by the target $\Delta F/F_0$ value,
which results in an optical closed-loop, and

(5) determining the feedback light parameters of step (4) and comparing them with the feedback light parameters determined for a control, wherein a difference between said feedback light parameters of step (4) and the control indicates that the mutation(s) of the ion channel and/or the added compound(s) have an effect on the ion channel, wherein the feedback light parameters are selected from the feedback wavelength(s), the course of the feedback wavelength(s) and/or the light intensity, and
wherein the control is the wildtype ion channel and/or no compound(s) are added.

The method of the present invention preferably further comprises a calibration and bleaching correction.
Said calibration and bleaching correction preferably comprises measuring the exponential decrease in fluorescence of the voltage indicator, such as a GEVI protein, and real-time correcting each incoming $\Delta F/F_0$ value accordingly.

The method of the present invention preferably further comprises a compensatory wavelength to counteract optical crosstalk.
Said which compensatory wavelength is preferably selected in case of (full) pre-activation of (one of) the tools and enables both the calculation of the starting fluorescence (Fo) as well as the bidirectional control / clamping of the membrane potential.

*Step (1) - cell with OVC and ion channcel*

In step (1), a cell is provided with comprises

the ion channel to be determined or modulated, and
a voltage indicator, and counteracting optogenetic protein(s) for depolarization and hyperpolarization.

The cell provided in step (1) can be any cell, in particular any cell in which a membrane potential is intrinsically determined or dynamically altered. It is a cell in which membrane potential is subject to dynamic changes or where changes in membrane potential affect membrane transport processes, for example through secondary active transporters.
In one embodiment, the vcell is an electrically excitable cell, such as a muscle cell, a neuron, a cardiomyocyte (such as iPSC-derived cardiomyocyte) or a glial cell.
The voltage indicator is a genetically encoded voltage indicator (GEVI) protein or a voltage sensitive dye.
Preferably, the GEVI protein is as defined herein. Voltage sensitive dyes are known in the art.
The cell provided in step (1) preferably co-expresses the ion channel, the GEVI and the counteracting optogenetic protein(s)
In one embodiment, the cell is a cell comprising the ion channel to be determined or modulated (such as a cell of a stable cell line expressing the ion channel) and a genetically encoded voltage indicator (GEVI) protein, and counteracting optogenetic protein(s) for depolarization and hyperpolarization are introduced.
In one embodiment, the cell is a cell according to the present invention, wherein furthermore the ion channel to be determined or modulated is introduced.
In one embodiment, the ion channel, a genetically encoded voltage indicator (GEVI) protein and counteracting optogenetic protein(s) for depolarization and hyperpolarization are introduced into the cell.
For determining the functionality of and/or for modulating ion channels and/or for determining influences on ion channels, respective conditions are also provided in step (1).
In one embodiment, ion channel variant(s) comprising mutation(s) are introduced into the cell. In one embodiment, compound(s) to be tested for an effect on the ion channel or its variant(s) are added.
In one embodiment, both ion channel variant(s) comprising mutation(s) are introduced into the cell and compound(s) to be tested for an effect on the ion channel variant(s) are added.

*Steps (2) and (3) -fluorescence of voltage indicator*

In step (2), the fluorescence of the voltage indicator, preferably a GEVI protein, is excited with a first light source.
The first light source is preferably a laser, an LED or an HBO lamp.
In step (3), the fluorescence emitted from the voltage indicator, preferably a GEVI protein, is recorded with a camera in real time.
From the intensity of fluorescence the relative change of fluorescence is determined ($\Delta F/F_0$).
The fluorescence recorded in step (3) is in linear dependence of the membrane voltage, i.e. depolarization results in increase of fluorescence, hyperpolarization results in decrease of fluorescence.
In one embodiment, in step (3) the camera records gray values and a software translates them into relative changes of fluorescence.

*Step (4) - controlling the membrane voltage through the counteracting optogenetic protein(s)*

Step (4) is carried out at the same time as steps (2) and (3), in parallel.
In step (4), the counteracting optogenetic protein(s) for depolarization and hyperpolarization are irradiated with a second light source.
The second light source is preferably a tunable light source, which can access a broad spectrum of wavelengths, but produces a narrow range of any of those wavelengths at a given time, such as a monochromator, a tunable laser.
Step (4) comprises the adjustment of a feedback wavelength of said second light source depending on (i) the difference between intended membrane voltage and present membrane voltage, reflected by relative changes in fluorescence ($\Delta F/F_0$),and (ii) the absorption maxima of the optogenetic protein(s).
Said feedback wavelength is adjusted

(a) towards the absorption maximum of the depolarizing tool, if the present membrane voltage is smaller than a target membrane voltage, whereas the membrane voltage is indicated by the relative change in fluorescence ($\Delta F/F_0$), and

(b) towards the absorption maximum of the hyperpolarizing tool, if the present membrane voltage is greater than a target membrane voltage, whereas the membrane voltage is indicated by the relative change in fluorescence ($\Delta F/F_0$),

to clamp the membrane potential at the target voltage, reflected by the target $\Delta F/F_0$ value, which results in an optical closed-loop.

As discussed above, the method preferably furthermore comprises one or more of the following:

(i) selecting a tolerance range for the difference between intended membrane voltage and present membrane voltage, reflected by relative changes in fluorescence ($\Delta F/F_0$), preferably 0.1 to 10%, more preferably 0.2 to 5%, such as 1%;

(ii) weighting of the feedback light depending on the size of deviation between intended membrane voltage and present membrane voltage, reflected by relative changes in fluorescence ($\Delta F/F_0$),

(iii) adapting the wavelength of the second light source in real time in increments, which depend on the size of deviation between intended membrane voltage and present membrane voltage, reflected by relative changes in fluorescence ($\Delta F/F_0$),as soon as the present $\Delta F/F_0$ value violates a selected tolerance range,

(iv) preselecting the target membrane voltage, as indicated by the relative change in fluorescence ($\Delta F/F_0$), or changing it in real time.

*Step (5) - Read out*

In step (5), the feedback light parameters of step (4) are determined and compared them with the light conditions determined for a control.

The feedback light parameters are selected from the feedback wavelength(s) and/or the course of the feedback wavelength(s) and/or the light intensity

The control is the wildtype ion channel and/or no compound(s) are added. The skilled artisan can determine which control conditions are suitable.

A difference between said feedback light parameters of step (4) and the control indicates that the mutation(s) of the ion channel and/or the added compound(s) have an effect on the ion channel.

The compound(s) to be tested for an effect on the ion channel or its variant(s) can be added at the beginning, in step (1) or later during the method, such as during steps (3) and (4).

The method is preferably suitable for ryanodine receptor, sodium channels, calcium channels, potassium channels (such as HERG K+ channel) and ligand-gated ion channels.
The method is also suitable for intracellular ion channels, such as ryanodine receptor.

*Further description of preferred embodiments*

In order to achieve true control over excitable cell activity in a living animal, we combined two opposing optogenetic actuators for de- and hyperpolarization and a genetically encoded voltage indicator with a software-based live closed-loop feedback system, inspired by voltage-clamp electrophysiology, in Caenorhabditis elegans. Being closed-loop, the system is responsive to the changing needs of the excitable cell tissue under consideration and circumvents the highly variable activity levels, triggered by conventional, static stimulation patterns. The system aims to synergize the non-invasive character of imaging methods with the control capabilities of electrophysiological recordings. For actuation, the OVC (optogenetic voltage-clamp) makes use of BiPOLES, a tandem protein consisting of the red-light activatable depolarizer Chrimson and the hyperpolarizer ACR2 that responds to green light (Bergs *et al.,* 2018; Vierock *et al.,* 2020; Govorunova *et al.,* 2015). Spectral separation and balanced 1:1 expression of BIPOLES enable gradual transitions from depolarized to hyperpolarized states and vice versa, making this tool first choice for bidirectional tuning of membrane voltage. For surveillance of voltage activity, QuasAr complements the OVC as a potent far-red voltage indicator that performed best in a side-by-side comparison study in C. elegans (Hochbaum *et al.,* 2014; Azimi Hashemi *et al.,* 2019). The voltage signals revealed by QuasAr are used to compute a fast feedback of light signals to BiPOLES, to keep the membrane potential at a desired holding fluorescence level that is selected by the experimenter.

To examine the performance of the OVC, we first established the system in easily accessible body-wall muscle cells, turned then to cholinergic and GABAergic motor-neurons and eventually tested its applicability in spontaneously active pharyngeal muscle and the GABAergic motor neuron DVB that activates expulsion muscle contraction during defecation (McIntire *et al.,* 1993; Avery and Thomas, 1997)

*- Abstract*

To date, optogenetics allows only perturbation, but no true control of neural activity. Therefore and inspired by voltage-clamp electrophysiology, we established an all-optical voltage clamp approach (OVC), in which fast (90 fps), live voltage readout is used to generate light-based feedback to optogenetic actuator tools, to clamp the cell at a distinct potential. To this end, we combined the optogenetic actuator pair BiPOLES with the GEVI QuasAr, embedded in a software-based live closed-loop feedback system. This way, the OVC synergistically combines the non-invasive character of imaging methods with the control capabilities of electrophysiological methods. We established our approach in muscle cells, cholinergic and GABAergic motor neurons of *Caenorhabditis elegans* and tested its applicability to dynamically clamp spontaneous spiking in the pharnyx and the GABAergic motor neuron DVB. Outperforming standard patch-clamp electrophysiology in terms of non-invasiveness, throughput and ease of application, the OVC paves the way for all-optical control of individual neurons in freely behaving animals.

*- Results*

*The OVC conducts clamping of QuasAr's fluorescence via a live closed-loop feedback system*

The development of the OVC involved three major parts, namely the optimization of interacting optogenetic actuators and voltage sensors in the worm, the arrangement of the hardware setup and the programming of a feedback-loop software, enabling the individual components to communicate.
The spectrum of optogenetic actuators is broad, e.g. varying in speed, spectral properties and ion-selectivity, the latter turning the tool into either a de- or hyperpolarizer (Govorunova *et al.,* 2015; Schultheis *et al.,* 2011; Dawydow *et al.,* 2014; Berndt *et al.,* 2009, Husson *et al.,* 2012). Spectrally balanced, counteracting protein partners are essential to guarantee for a smooth regulation of membrane polarization (Figure 1A). For observation of voltage dynamics, we complemented the system with a far-red voltage indicator (Figure 1A) that is stimulated by a 637 nm laser. In addition to the laser, the hardware setup (Figure 1B) consists of a highly sensitive EMCCD camera that allows recording at high framerates despite the low absolute fluorescence of rhodopsin-based GEVIs. To ensure a flexible stimulation of optogenetic actuators that can keep up with the camera's speed and the voltage signal dynamics to be observed, we added a monochromator acting at a temporal and spectral resolution of 100 μs and 0.1 nm, respectively. The communication between monochromatic light source and camera is provided by a custom written script for the open source microscope control software Micro-Manager. Our software performs live processing of incoming gray values from the camera, translating them into relative changes of fluorescence ($\Delta F/F_0$),based on a user-selectable calibration phase (Figure 1C). Here, computation allows a maximum speed of around 90 fps (10 ms exposure). Since rhodopsin-based voltage sensors suffer from unneglectable photobleaching, which would falsify the actual voltage levels, an initial calibration phase is used to evaluate the exponential decay for bleaching correction - live during the running acquisition (Equations (1) to (3) below; Miura *et al.,* 2014). Once the system has access to the bleaching-corrected $\Delta F/F_0$ values, it feeds them into a simple decision tree algorithm, where they are compared to a desired holding $\Delta F/F_0$ value. The deviation between command and current relative change in fluorescence determines the wavelength change of the monochromator (Equation (5) below).
The algorithm reacts with adaptable wavelength increments via a multiplier element (mirroring the difference to the holding value), which accelerates the adaptation process (Equation (6) below). The main software comes with a three-step protocol, where among others, the desired holding values (within a tolerance range) and number of frames for each step can be selected by the user. To save computation capacity, simple rectangular ROIs are selected prior to the experiment instead of polygon shapes.

$$\text{exp. offset} = a \times e^{-b \times frame} + c \qquad (1)$$

$$\text{ratio} = \text{exp. offset}_0 \div \text{exp. offset}_{frame} \qquad (2)$$

$$Fc_{orrect} = F_{frame} \times \text{ratio} \qquad (3)$$

$$\Delta F/F_{0\,correct} = ((F_{correct} - F_0) / F_0) * 100 \qquad (4)$$

$$multiplier = |\Delta F/F_{0\,holding} - \Delta F/F_0| \qquad (5)$$

$$wavelength = wavelength_{frame-1} + multiplier * increment \qquad (6)$$

exp. offset: Exponential offset. a, b, c: Exponential fitting parameters. ratio: Ratio between first and current exponential offset. $F_{correct}$: Bleaching-corrected gray values. $F_{frame}$: Gray value of current frame. $\Delta F/F_0$ *Correct:* Bleaching-corrected relative change in fluorescence. Fo: Resting fluorescence value. Equations (1)-(3) based on the ImageJ Plugin "Correctbleach" (Miura *et al.,* 2014).

*Single-actuator combination with QuasAr enables unidirectional clamping of membrane voltage*

For initial software testing we investigated already established voltage imaging strains, that combine QuasAr in body wall muscle (BWMs) with either ChR2(H134R) or ACR2 expression in cholinergic motor-neurons (Azimi Hashemi *et al.,* 2019) (data not shown). Here, the membrane potential in QuasAr expressing BWMs is indirectly manipulated via light-induced (de-)activation of upstream innervating motor-neurons. Calibration phases of around 20 s turned out to be sufficient to estimate the parameters of the exponentially decaying bleaching behavior (data not shown). In QuasAr-only expressing animals, blue light of 300 μW/mm2, had no obvious influence on bleaching behavior compared to red laser light only excitation during calibration (Figure 3D). In the same strain, conducting a clamping protocol from -5 to 5 % $\Delta F/F_0$ had no impact on QuasAr's fluorescence and therefore failed as expected (data not shown).

With ChR2 activation, QuasAr's fluorescence could be reliably clamped to values between 0 - 20 % ($\Delta F/F_0$, while ACR2 enabled to clamp the fluorescent signal to as low as -15 % ($\Delta F/F_0$ (data not shown). However, for both tool combinations, returning to the baseline and counteracting of overshoots in the respective direction are only based on rather slow intrinsic membrane potential relaxation of the cells (data not shown). This restricts the system's temporal resolution (ChR2: 150 ± 12.7 ms to reach tolerance range and 680 ± 14.9 ms back to baseline; ACR2: 254 ± 20.4 ms and 240 ± 30 ms respectively) (data not shown) and leads in some cases to a persistent exceeding of the selected $\Delta F/F_0$ tolerance range (20 % $\Delta F/F_0$ trace, data not shown).
This demonstrates the importance of an opposing optogenetic tool as it is realized in the present invention.

*The OVC enables bidirectional adjustment of membrane potential in BWMs, cholinergic and GABAergic neurons*

Initial testing of spectrally distinct actuation pairs like ChR2 (peak absorption: 470 nm) and ACR1 (peak absorption: 515 nm), expressed as individual proteins in different relative concentrations failed due to barely controllable expression levels, where one of the components was typically outperformed by the other (data not shown). Expression level issues are overcome by using the tandem protein BiPOLES, in which an additional transmembrane helix links the highly efficient depolarizer Chrimson (peak absorption: 590 nm) to the equally efficient chloride-selective hyperpolarizer ACR2 (peak absorption: 470 nm) (Vierock *et al.,* 2020). With BiPOLES, the OVC makes use of a tandem tool allowing bidirectional control, thus overcoming the disadvantages of single-tool experiments presented above.

We combined expression of QuasAr with BiPOLES in BWMs in a same-cell approach (Figure 2A) after initially investigating the tools in a transsynaptic configuration (BiPOLES expressed in cholinergic neurons, QuasAr expressed in BWMs), for better comparison with the original single-tool combinations. Expression could be detected by mCerulean fused to BiPOLES, localized to BWMs (same-cell), and QuasAr's fluorescence at 700 nm emission, localized to the plasma membrane of BWMs (Figure 2B). To circumvent the inevitable pre-activation of Chrimson by red laser light, we used a monochromator calibration wavelength for compensation of optical crosstalk. In this context, body-length measurements of BIPOLES expressing animals (chol. neurons) revealed normal motor behavior at the transition point where hyperpolarization, accompanied by body relaxation, merged into depolarization that triggers body contraction (Figure 2C). This wavelength was successively adjusted with the first animals of the experimental series until the full range of -5 to 5 % $\Delta F/F_0$ in the standard protocol could be maintained for each of the 5 s steps. Increment-wise changes in wavelength closely followed the fluctuating fluorescence signals, as soon as the tolerance range for holding $\Delta F/F_0$ was exceeded or the system reached a transition point (Figure 2E). The OVC allowed reliable clamping to holding values between -5 and 5 % $\Delta F/F_0$, for some animals even a broader range of -8 to 8 % $\Delta F/F_0$ (Figure 2D, F and H). This maximum range lags behind that of single-tool experiments, since the action spectra of Chrimson and ACR2 are partly overlapping. Due to the bidirectional control options with BiPOLES, the OVC acted significantly faster than single-tool approaches, which is reflected by the transition time needed for each step of the standard protocol (single-tool/ OVC BWMs (119.02 ± 9.3 ms): ***p = 1.77E-5, Figure 3A). Moreover, the OVC allowed continuous clamping (in the minute range) in both directions (Figure 3B). Once the script was interrupted, the membrane voltage relaxed back to the baseline and higher fluorescence fluctuations could be observed (Figure 3C).

For rigorous characterization, we determined the actual membrane voltages that the OVC-controlled voltage fluorescence levels correspond to. To enable better comparability, we first performed wavelength ramp experiments in the OVC configuration, sliding from 400 to 600 nm and *vice versa,* to investigate the maximum range of relative fluorescence changes (Figure 2 F). This revealed a maximum range of around 16 % $\Delta F/F_0$ (difference of fluorescence levels achieved with 401 - 410 nm and with 590 - 600 nm monochromator light, Figure 2 H, upper panel), which is in line with the maximum range of holding values stably achieved with the OVC ($\pm$ 8 % $\Delta F/F_0$, compare Figure 2I). In current clamp electrophysiology, at corresponding wavelengths, we observed a range of around 18 mV in BWM cells (Figure 2 G and H, lower panel). Since the fluorescence-voltage relation of QuasAr is linear, we estimate that during the typical OVC protocol (-5% to +5% $\Delta F/F_0$), about 11.25 mV change in membrane potential is achieved.

To extend the applicability of the OVC, a further software version was developed that allows to select holding $\Delta F/F_0$ values in 1 % steps live, during a running acquisition (Figure 2 I). This additional approach facilitates a direct reaction to the experimenter's observation and allows to dynamically adapt rather than pre-select clamping parameters. Therefore, the software provides a live status update, whether the system is on hold, adapting or if it has reached its wavelength limits, whereas the latter indicates a system saturation.

To further demonstrate the usefulness of the OVC, we compared wild-type with *unc-13(n2813)* mutant animals (synaptic vesicle priming factor) (Richmond *et al.,* 1999). A previous study showed drastically reduced ChR2-induced photo-currents for this and other mutants with cholinergic synaptic defects, but enhanced muscle contractions in behavioral experiments (Liewald *et al.,* 2008). To shed light on this apparent contradiction, we performed OVC measurements geared to observe depolarized states (Figure 4 A and B). Interestingly, we found that during the 5 % $\Delta F/F_0$ step, the OVC counteracted with significantly less red-shifted wavelengths in *unc-13(n2813)* animals compared to the wild-type (wild-type (550.4 $\pm$ 3.1 nm)/ *unc-13(n2813)* (534.2 $\pm$ 2.8 nm): **p = 0.0012). This means that less activation of Chrimson was required to induce the same level of depolarization as in the wild-type, which indicates a higher muscle excitability in the mutant and resolves the addressed paradoxical relation between currents and muscle contraction.

After confirmation of the OVC's functionality in the large and thus easily accessible (i.e. providing large absolute fluorescence amplitudes) BWMs, we turned to achieve a same-cell approach also in cholinergic and GABAergic motor neurons (Figure 5A), that are more challenging to observe due to their comparably small size. As for the BWM strains, fluorescence of both mCeruelean (BiPOLES) and QuasAr was localized to the respective neuronal tissue, including the nerve ring in the head part of the animals, as well as along the ventral nerve cord (Figure 5B). In line with experiments in muscle, the OVC succeeded to clamp the neurons' fluorescence between -5 to 5 % $\Delta F/F_0$ (Figure 5D and E). The calibration phase parameters could directly be adopted from the experiments in muscles (Figure 5C). Again, the OVC performed significantly faster compared to single-tool experiments in BWMs (single-tool/ OVC chol. neurons (190.6 $\pm$ 18.9 ms): **p = 0.008, single-tool/ OVC GABAergic neurons (174.9 $\pm$ 16.1 ms): *p = 0.037, Figure 3A). "On-the-run" selecting of holding $\Delta F/F_0$ values was also applicable to cholinergic and GABAergic neurons (Figure 5 F and G), where it allowed to react to when the system was getting close to saturation, i.e. its wavelength limits during the experiment (Figure 5 F and G).

*The OVC allows to specifically suppress action potentials in spontaneously active pharyngeal muscle and the GABAergic motor neuron DVB*

To test whether the OVC system is also capable to counteract and suppress distinct voltage events, especially action potentials (APs), we looked at the pharynx, a spontaneously active muscular pump used by C. *elegans* for feeding and the GABAergic motor neuron DVB that shows regular APs during the defecation motor program (Figure 6 A and J). In both cases, APs occur prior to rhythmic muscular contractions.

Pharyngeal APs can be triggered by incubating the animals in serotonin, resulting in high frequency AP trains. At the behavioral level, pumping is accompanied by the opening and closing of the grinder, which could be triggered by activation of the ACR2 component of BiPOLES (400 nm light), while observing QuasAr's fluorescence. When ACR2 was activated, fluorescence immediately dropped, followed by the closing of the grinder. Contrastingly, exclusive laser illumination at 637 nm was associated with opening of the grinder. As for the other tissues, also in the pharynx, the OVC allowed clamping between -5 to 5 % $\Delta F/F_0$, and "on-the-run" selection of holding $\Delta F/F_0$ values (Figure 6 B and C).

Optically observed pharyngeal APs show a rise time constant of around 15 ms and a total AP duration of $\approx$ 250 ms18. To clamp these APs, i.e. to counteract de- and repolarization phases fast enough, it was necessary to respond with pronounced wavelength changes. In this software version, the wavelength change is determined by the difference between holding- and current $\Delta F/F_0$ values, amplified by a multiplier element, so that stronger feedback can be generated in less time (Equation (6)). To increase the algorithm's sensitivity, the tolerance range was reduced down to $\pm$ 0.005 %, to ensure that each violation of the tolerance range limit was followed by a wavelength adaptation. During the calibration phase of the experiments, we observed several AP trains with a mean amplitude of 30.1 $\pm$ 1.1 % $\Delta F/F_0$ and duration of 0.12 $\pm$ 0.004 s at FWHM (Figure 6 D, E, H and I). As soon as the system switched to the clamping state, all subsequent attempts of the pharynx to spike were counteracted and almost completely suppressed to around 10 % of the initial amplitude (Figure 6D-I). The mean AP amplitude (spontaneous/clamped (2.9 ± 0.2 % $\Delta F/F_0$): ***p = 2.2E-25) and duration (spontaneous/clamped (0.08 ± 0.003 s): ***p = 5.9E-8) were significantly reduced for the clamped events (Figure 6I). In the example shown in Figure 6D, one AP at ca. 20.3 s, right when the clamping phase began, showed an aberrant overall appearance with ca. 35 % duration (at FWHM) of the preceding APs. To counteract this AP, the wavelength spectrum (i.e. 600 nm to 400 nm) was fully traversed within 20 ms (Figure 6G).

In C. *elegans,* the expulsion muscle contraction is regulated by the GABAergic motor neurons DVB and AVL. In line with previous studies, that employ $Ca^{2+}$-Imaging to study neural activity (Wang *et al.,* 2013), voltage imaging in DVB revealed the occurrence of APs in regular time intervals of around 40 to 50 s (Figure 7 B). Spontaneous AP voltage signals in DVB showed a mean amplitude of 7.7 ± 1 % $\Delta F/F_0$ and duration of 0.9 ± 0.03 s (Figure 7 B and D). To counteract those APs, we used the same configurations of the OVC software as for the optical clamping of pharyngeal muscle, described above. In doing so, we observed a significantly reduced mean AP amplitude (spontaneous/clamped (3.1 ± 0.6 % $\Delta F/F_0$): **p = 0.007) and duration (spontaneous/clamped (0.2 ± 0.05 s): ***p = 7.9E-8) for the clamped events (Figure D).

*Evaluation and electrophysiological calibration of the OVC*

For evaluation of the OVC, we investigated the quality of the exponential fit for bleach correction (coefficient of determination $R^2$), the respective percentages of the clamping status (hold, adapting, system failure), the system's speed reflected by the time required for a 5 % $\Delta F/F_0$ step (Figure 3A).

In general, the predictive accuracy for bleaching correction was high with $R^2$ values above 0.7 (data not shown). For some experiments $R^2$ fell below this value, since more action potentials occurred during the calibration phase (e.g. OVC in pharyngeal muscle). However, the occurrence of APs had no obvious impact on the success of the OVC recording. Those measurements with $R^2$ values below 0.7, which were unrelated to action potentials, e.g. when the animal was moving, were excluded from further evaluation. As expected, the fraction of system saturation decreased once optogenetic tools for de- and hyperpolarization were simultaneously used, which stresses the indispensability of a second opposing actuator (data not shown). While for the single-tool approaches, the percentage of system saturation was relatively high with 16.3 ± 2.8 % (although the clamping was only performed in the preferred direction of the respective tool), this relation was significantly reduced for all bidirectional BiPOLES combinations (single-tool/ BWMs transsynaptic (3.6 ± 1.5 %): **p = 0.0072, single-tool/ OVC BWMs same-cell (5.9 ± 2.1 %): *p = 0.043, single-tool/ OVC pharnyx (2.4 ± 1.3 %): **p = 0.0013, single-tool/ OVC chol. neurons (0.4 ± 0.4 %): ***p = 5.2E-4, single-tool/ OVC GABAergic neurons (3.9 ± 1.5 %): *p = 0.049). The same correlation was also reflected in the speed of the system (Figure 3A). For combinations of ChR2 or ACR2 with QuasAr, a 5 % step took on average 339 ± 50 ms, whereas OVC in muscle acted almost three times as fast with 119.02 ± 9.3 ms. Compared to single-tool combinations, all BiPOLES approaches were significantly faster (single-tool/ BWMs separated (104.5 ± 10.2 ms): ***p = 2.5E-6, single-tool/ OVC BWMs (119.02 ± 9.3 ms): ***p = 1.77E-5, single-tool/ OVC pharnyx (198.2 ± 16.1 ms): *p = 0.0102, single-tool/ OVC chol. neurons (190.6 ± 18.9 ms): **p = 0.008, single-tool/ OVC GABAergic neurons (174.9 ± 16.1 ms): *p = 0.037).

*- Discussion*

With the OVC, we established the first all-optical voltage clamp approach to date and demonstrated its performance in various excitable cells of intact *C. elegans.* For all tissue types, the OVC allowed reliable clamping of QuasAr's fluorescence, directly mirroring the cells' membrane voltage. Although orchestrating the communication between several hardware components, while in parallel running an image acquisition and providing live computation of bleaching-corrected $\Delta F/F_0$ values fed into a decision tree algorithm, the OVC acted fast, at framerates of up to 90 fps on a typical PC with moderate computing power. While falling behind standard patch-clamp electrophysiology with respect to speed and sensitivity, our method outperforms it in terms of non-invasiveness, throughput and ease of application. It even paves the way for a tight, all-optical control of individual neurons in freely behaving animals like C. *elegans,* and fine tuning of behavioral aspects.

The OVC described herein was optimized for the nematode, it can be easily transferred to other model organisms or cell culture, that allow for BiPOLES and QuasAr expression. The software component is universally applicable, as it can be easily adapted to other and to future GEVI-optogenetic actuator combinations and, by running on the open-access platform Micro-Manager, provides comprehensive hardware support and allows custom script editing to meet the requirements of individual future experiments.

The OVC system enriches the applicability of optogenetics as it allows neural modulation in a tightly controlled closed-loop configuration instead of a purely static optical perturbation that cannot meet the demands of highly variable activity patterns found in living organisms.

Dynamical responsiveness is particularly advantageous with regard to therapeutic applications of optogenetics, as it allows to adapt the therapy to the patient's needs.

*Screening of ion channels and/or drugs*

*- Mutagenesis screening of ion channels*

The OVC method of the present invention is suitable to perform high-throughput mutagenesis screens of ion channels. This is of interest for ion channel structure-function analyses to improve the understanding of the role of individual amino acids for the function of a particular ion channel. Classically, such information is obtained from 1) mutagenesis studies in animal models, where mutations with interesting properties are isolated. However, this requires that the mutation has very strong effects that are reflected macroscopically, e.g., in the movement or other behavior of the animal. Mutations with subtle effects are usually not noticed, and if the channel has no influence on macroscopic effects anyway, it is difficult to be addressed by forward genetic screens. In option 2), one already knows the structure of the corresponding channel and can then generate and test specific mutations. However, for this option one must already have a good idea, which amino acid position might be of particular interest. Unexpected things will not necessarily be found this way. Both approaches are very laborious and timeconsuming.

With the help of the OVC technique of the present invention, mutagenesis screenings of ion channels can be enormously facilitated: First, the optogenetic tools for de- and hyperpolarization (e.g. BiPOLES) and the voltage sensor (e.g. QuasAr) required for the OVC technique are introduced into a cell line via a stable transfection (permanent integration into the genome) - this cell line is then available for all further experiments. The optogenetic tools are expressed in optimized amounts so that their summed conductance is sufficient to compensate the currents that an additional ion channel (which is to be investigated). The components of BiPOLES are currently the most powerful light-activated ion channels known, and the expression level of the ion channel under investigation is adjusted accordingly. For the actual screening, cells of the generated OVC cell line are transfected with randomly mutated versions of an ion channel (plasmid library, e.g. generated by error-prone PCR). This results in single cells, each expressing a different mutant of the channel. These can be cultured either diluted in large culture dishes such that they are visible individually or as colonies. Alternatively, individual cells can be distributed by a FACS device into individual wells of a microtiter plate and further expanded (clonal expansion). An automated system is then used to scan these cells / microtiter wells, with a camera and the necessary light sources (e.g. laser and monochromator) moving from cell to cell and performing an OVC measurement. For the OVC measurement, any fluorescence-based clamp protocol is conceivable, which can be adapted to the ion channel under investigation. For the length of the measurement, photobleaching of the voltage sensor must be taken into account, as this could falsify the fluorescence data for longer measurements. In the OVC system of the present invention, this possible falsification is counteracted by a calibration phase preceding the experiment (duration: 20 s). For very short measurements (in the range of one second), this initial calibration can be omitted if necessary, which increases the throughput of the technique. Another possibility is to extrapolate the parameters of the exponential fit on the basis of a test series and to apply these parameters to all further measurements, which also makes the calibration phase unnecessary. Using the OVC technique of the present invention for this application, makes it possible to identify interesting variants (mutants) of the ion channel and subsequently isolate and sequence them.

In *C. elegans,* we have already detected differences between the wild type and a synaptic transmission mutant (*unc-13*) using the OVC technique, as described herein, which was manifested by a significantly different excitability of the tested cells (feedback light wavelengths were significantly altered).

*- High-throughput drug screening*

Another application of the OVC method of the present invention (based on the application mentioned above), is high-throughput drug screening. Various pharmaceutical companies already have large libraries of pure compounds (in some cases several million compounds) that could be potential lead compounds for specific interactions with certain ion channels. They just have to be found. To do this, cells of the pre-generated OVC cell line is first transfected with a corresponding ion channel (e.g. an L-type $Ca^{2+}$ channel or the HERG channel, which have characteristic mutations in certain heart diseases) and cultured in a microtiter plate. To each of these wells a different test substance from a so-called "compound library" is added. The OVC measurement is performed using a high-throughput plate reader (in 384-well format). Such an automated device has a sensitive and fast camera (or photomultiplier), as well as the two light channels to excite the optogenetic tools and the voltage sensor.

Assuming a measurement duration of a few seconds (if the calibration phase can be skipped), the 384 compounds are tested within a few minutes. Due to the purely optical character of the OVC, this application can be performed much easier and possibly more cost-efficiently than with an automated patch clamp system (e.g. https://sophion.com/products/qpatch-automated-patch-clamp/), since it works contactless and without pipettes and only smaller volumes are needed. Furthermore, by using the OVC technique of the present invention, cell-to-cell variability is more negligible, since the optical measurement averages the effect of hundreds of cells per substance, unlike the patch-clamp method where only a single cell can be observed (patched).

*- Outcome parameters / course of a measurement*

Depending on the properties of the ion channel (e.g. voltage-gated or constitutively open), effects occur at a specific membrane potential. The conductivity or the gating properties of the channel, if they are affected by a substance or a mutation, then also have an effect on the measurement protocol, since the OVC system has to set different wavelengths (feedback wavelength courses) to counteract the current of the channel. Differences in feedback wavelengths (or feedback wavelength courses) between the wild-type ion channel and the mutant variants, or ion channels affected by a substance, then indicate mutants and substances of interest.

The following examples and drawings illustrate the present invention without, however, limiting the same thereto.

BRIEF DESCRIPTION OF THE DRAWINGS

**Figure 1**. *Components, setup and functionality of the optogenetic voltage clamp (OVC) according to the present application.*

**(A)** Scheme of molecular OVC components, consisting of two counteracting tools for de- and hyperpolarization and a voltage indicator.
**(B)** Scheme of hardware setup and component communication: Membrane voltage is monitored via a camera and incoming grey values are processed by a live control software at each timepoint. Based on the deviation to the holding value, a feedback of light is sent back to the optogenetic actuators via a monochromatic light source.
**(C)** Feedback mechanism of the OVC: After selection of clamping parameters and calibration for bleaching correction, $\Delta F/F_0$ is calculated at each time point and compared with the preselected holding value. Then, the wavelength is adapted accordingly.

**Figure 2.** *Optogenetic bi-directional clamping of membrane voltage in BWMs.*

(A) Expression scheme of the OVC in BWMs (*C. elegans*)*:* Animals express both BiPOLES (tandem protein consisting of ACR2 and Chrimson) and QuasAr in BWMs. Fused mCerulean (fluorescent marker protein) depicted as star (★).
**(B)** Fluorescence images of BiPOLES and QuasAr expression. Scale is 50 $\mu$m.
**(C)** Example trace of the OVC three-step protocol in BWMs (same-cell). Left panel: Raw and bleaching corrected data of calibration and clamping phase. Right panel: Corresponding wavelength and $\Delta F/F_0$ traces. Holding values were 0, -5 and 5 % $\Delta F/F_0$. Holding values and tolerance range as indicated for each step.
**(D)** Upper panel: Overlay of wavelength and fluorescence traces. Lower panel: Magnification of upper panel to demonstrate fast transition between the steps. Transition phase highlighted in dashed box (time needed to reach tolerance range).
**(E)** Mean traces of the OVC in BWMs (same-cell) of the standard protocol (n = 22, holding values: 0, -5, 5 % $\Delta F/F_0$).
**(F-H)** Electrophysiological calibration measurements in BWMs
**(F)** Relative changes in fluorescence ($\Delta F/F_0$) upon presenting a wavelength ramp from 400 to 600 nm (upper panel) and vice versa (lower panel) (simultaneous laser excitation). **(G)** Membrane potential recorded in whole-cell patch-clamp mode, while patched cells were stimulated by the same light protocols as in (F) (simultaneous laser excitation). **(H)** Upper panel: Mean relative fluorescence change between 401 and 410 nm, and 590 and 600 nm of the data presented in (F). Lower panel: Mean membrane potential between 401 and 410 nm, and 590 and 600 nm of the data presented in (G).
**(I)** "On-the-run"-mode in muscle cells. Holding values were selected live during the measurement.

Insert: Optical clamping parameters can be selected live, during the running experiment (indicated by arrow keys of a computer keyboard). Live status update (system on hold, wavelength adapting, or wavelength limits reached) is presented as a decision basis for further adjustment of the clamping value (visualized by traffic light symbol).

**Figure 3.** *OVC control experiments and evaluation.*

**(A)** Summary of the system's speed reflected by the time needed for a 5 % $\Delta F/F_0$ step. Statistically analyzed by one-way-ANOVA with Bonferroni correction. ***p $\leq$ 0.001, **P $\leq$ 0.01, *P $\leq$ 0.05.
**(B)** Long-term OVC experiment (BWMs: BiPOLES and QuasAr, holding values as indicated).
**(C)** Left panel: OVC experiment in BWMs (BiPOLES and QuasAr) with clamp interruption (holding values: -5, interruption at calibration wavelength, -5 % DF/F0). Right panel: Magnification of (C) with comparison to the voltage activity of unstimulated QuasAr-only expressing animals.

**(D)** Mean traces of the OVC standard protocol in BWMs of QuasAr-only expressing control animals (n = 8, holding values: 0, -5, 5 % ΔF/F0).

**Figure 4.**

**(A)** OVC measurement (mean traces) of *unc-13(n2813)* mutant animals in comparison to wildtype (n = 9-10, holding values: 0, 5, 0 % $\Delta F/F_0$).

**(B)** Statistical analysis of (A). Mean wavelength of the second step (5 % $\Delta F/F_0$).Statistically analyzed byone-way-ANOVA with Bonferroni correction. ***P ≤ 0.001, **P≤ 0.01, *P ≤ 0.05.

**Figure 5.** *Optogenetic bi-directional clamping of membrane voltage in neurons.*

(A) Expression scheme of the OVC in cholinergic or GABAergic neurons.

**(B)** Fluorescence images of BiPOLES and QuasAr expression. Scale is 50 μm.

**(C)** Example trace of three-step protocol of the OVC in cholinergic neurons. Holding values were 0, -5 and 5 % $\Delta F/F_0$.

**(D)** Mean traces of the OVC in cholinergic neurons of the standard protocol (n = 14, holding values: 0, -5, 5 % $\Delta F/F_0$).

**(E)** Mean traces of the OVC in GABAergic neurons of the standard protocol (n = 7, holding values: 0, -5, 5 % $\Delta F/F_0$).

**(F)** "On-the-run"-mode experiment of the OVC in cholinergic neurons. Holding values were selected live during the measurement. Experimenter reacted to reaching the lower wavelength limit (highlighted).

**(G)** "On-the-run"-mode experiment of the OVC in GABAergic neurons.

**Figure 6.** *Suppression of spontaneous APs in the pharynx.*

**(A)** Scheme of the pharynx with respective fluorescence image of QuasAr expression. Scale is 50 μm.

**(B)** Mean traces of the OVC standard protocol in the pharnyx (n = 16, holding values: 0, -5, 5 % $\Delta F/F_0$).

**(C)** "On-the-run"-mode experiment of the OVC in the pharynx. Holding values were selected live during the measurement.

**(D)** Example trace of the OVC in the pharynx to hold fluorescence at 0% $\Delta F/F_0$ and suppress APs with a multiplier of 4x.

**(E)** Magnification of (D) to show transition between calibration and clamp phase.

**(F)** Overlay of wavelength and $\Delta F/F_0$ traces during clamp phase.

**(G)** Magnification of (F) to show the OVC counteracting the first AP during clamp phase.

**(H)** Overlay of spontaneous and clamped pharynx voltage signals. (spontaneous: n = 20, clamped: n = 20).

**(I)** Statistical analysis of (H), showing the mean voltage signal amplitude and duration at FWHM. Statistically analyzed by one-way-ANOVA with Bonferroni correction. ***P ≤ 0.001, **P ≤ 0.01, *P ≤ 0.05.

**Figure** 7. *Suppression of spontaneous APs in the GABAergic motor neuron DVB.*

**(A)** Scheme DVB with respective fluorescence image of QuasAr expression. Scale is 50 μm.

**(B)** Voltage imaging of spontaneous APs in DVB (blue trace). Right panel: Magnification. Expulsion muscle contraction depicted in grey.

**(C)** Overlay of spontaneous and clamped DVB voltage signals. (spontaneous: n = 8, clamped: n = 5).

**(D)** Statistical analysis of (C), showing the mean voltage signal amplitude and duration at FWHM. Statistically analyzed by one-way-ANOVA with Bonferroni correction. ***P ≤ 0.001, **P ≤ 0.01, *P ≤ 0.05.

**(E)** Patch clamp calibration recordings in DVB, in dissected animals.

**(F)** Statistical analysis, showing the resting potential, threshold, peak amplitude, the duration at FWHM and afterhyperpolarization (AHP) (n=5).

## EXAMPLES

### Example 1 Material and Methods

#### 1. *C. elegans* maintenance and transgenic animals

Animals were cultivated on nematode growth medium (NGM) plates, seeded with E. coli OP50-1 strain (Liewald *et al.,* 2008). Generation of transgenic animals was performed by microinjection of plasmid DNA, at varying concentrations (Evans, 2006). For transgenic strains ZX2476, ZX2482, ZX2483, ZX2714, ZX2753 and ZX2828, 10 ng/μL of the QuasAr voltage sensor plasmid DNA was injected into the gonads of young adult hermaphrodites, while 50 ng/μL were used for strain ZX1268, 1.5 ng/μL for ZX2826 and 50 ng/μL for ZX2827. BiPOLES plasmid DNA was injected at a concentration of 30 ng/μL for strains ZX2586, ZX2714, ZX2753 and ZX2827, whereas 1.5 ng/μL were injected for ZX2826 and 50 ng/μL for ZX2828. For strain ZX2483 50 ng/μL of ACR2 plasmid DNA were injected. Tools were combined with either 1.5 ng/μL of the co-expression marker pmyo-2::CFP, 30 ng/μL of pelt-2::GFP or 40 ng/μL of pmyo-3::CFP.

#### 2. Transgenic *C. elegans* strains

The following strains were used or generated:

| | |
|---|---|
| **ZX2476:** | *zxEx1139[pmyo-3::QuasAr; pmyo-2::CFP],* |
| **ZX2482** : | *zxEx1145[pmyo-3::QuasAr; pmyo-2::CFP]; zxIs5[punc-17::chop2(H134R)::yfp; lin-15+],* |
| **ZX2483** : | *zxEx1146[punc-17::ACR2::eYFP; pmyo-3::QuasAr; pelt-2::GFP],* |
| **ZX2586** : | *:GFP], zxEx1228[punc-17::GtACR2::mCerulean::bHK::Chrimson; pELT-2:* |
| **ZX2714:** | *zxEx1250[punc-17::GtACR2::mCerulean::bHK::Chrimson;pmyo-3: :QuasAr; pELT-2::GFP],* |
| **ZX2753:** | *zxEx1266[pmyo-3::GtACR2::mCerulean::bHK::Chrimson; pmyo-3: :QuasAr; pmyo-2::CFP],* |
| **ZX1268:** | *zxEx1268[punc-47::QuasAr::GFP; pmyo-2: :CFP],* |
| **ZX2826:** | *zxEx1282[pmyo-2::QuasAr; pmyo-2::GtACR2::mCerulean::bHK: :Chrimson; pmyo-3::CFP],* |
| **ZX2827:** | *zxEx1283[punc-17::GtACR2::mCerulean::bHK::Chrimson; punc-17, :QuasAr; pELT-2::GFP],* |
| **ZX2828** : | *zxEx1284[punc-47::QuasAr::GFP; punc-47: :GtACR2: :mCerulean: :bHK::Chrimson; pmyo-2::CFP],* |
| **ZX2876:** | *zxIs139[pmyo-3::GtACR2::mCerulean::bHK::chrimson;pmyo-3: :QuasAr; pmyo-2::CFP],* |
| **ZX2935:** | *unc-13(n2813);zxIs139[pmyo-3::GtACR2::mCerulean::bHK: :Chrimson; pmyo-3::QuasAr; pmyo-2:: CFP].* |

#### 3. Molecular biology

Plasmids pAB4 (*punc*-17::ACR2::eYFP), pAB16 (*pmyo*-3::QuasAr), pAB17 (*punc*-17::QuasAr), pAB23 (*ptdc*-1s::QuasAr::GFP) and pNH12 (*pmyo*-2::MacQ::mCitrine) were described earlier (Bergs *et al.,* 2018; Azimi Hashemi *et al.*,2019).

pAB26 (*punc*-17::GtACR2::mCerulean::bHK::Chrimson) was generated via Gibson Assembly based on RM#348p (*punc-17;* Zhang *et al.,* 2007; Liewald *et al.,* 2008) and pAAV-hSyn-BiPOLES-mCerulean (Addgene #154944), using restriction enzyme NheI and primers punc-17_ACR2_Chrimson_fwd (5'-attttcaggaggacccttggATGGCATCACAGGTCGTC-3') [SEQ ID NO. 1], and punc-17_ACR2_Chrimson_rev (5'-ataccatggtaccgtcgacgTCACACTGTGTCCTCGTC-3') [SEQ ID NO. 2].

pAB27 (pmyo- 3::GtACR2::mCerulean::bHK::Chrimson) was generated via Gibson Assembly based on pDD96.52 (*pmyo-3,* Addgene plasmid #1608) and pAAV-hSyn-BiPOLES-mCerulean, using restriction enzyme BamHI and primers acr2+chrimson _fwd (5'-actagatccatctagagATGGCATCACAGGTCGTC-3') [SEQ ID NO. 3]and acr2+chrimson_rev (5'-ttggccaatcccgggCACTGTGTCCTCGTCCTC-3') [SEQ ID NO. 4].

pAB28 (*punc*-47::QuasAr::GFP) was generated via Gibson Assembly based on pMSM08 (*punc*-47::eGFP::MmBoNTB) and pAB23 (*ptdc*-1s::QuasAr::GFP), using the restriction enzymes XmaI and MscI and primers punc-47_QuasAr_GFP_fwd (5'-ttacagcaccggtggattggATGGTAAGTATCGCTCTG-3') [SEQ ID NO. 5] and punc-47_QuasAr_GFP_rev (5'-ttctacgaatgctcctaggcCTATTTGTATAGTTCATCCATGC-3') [SEQ ID NO. 6].

pAB29 (*pmyo*-2::QuasAr) was generated via Gibson Assembly based on pNH12 (*pmyo*-2::MacQ::mCitrine) and pAB16 (pmyo-3::QuasAr), using primers pmyo-2QuasAr _fwd_vector (5'-caccgagtgaGAAGAGCAGGATCACCAG-3') [SEQ ID NO. 7], pmyo-2QuasAr_rev_vector (5'-tgcagagcgatacttaccatCCCCGAGGGTTAAAATGAAAAG-3') [SEQ ID NO. 8], pmyo-2QuasAr_fwd_insert (5'-ATGGTAAGTATCGCTCTGCAG-3') [SEQ ID NO. 9] and pmyo-2QuasAr_rev_insert (5'-cctgctcttctcaCTCGGTGTCGCCCAGAATAG-3') [SEQ ID NO. 10].

pAB30 (*pmyo*-2::GtACR2::mCerulean::bHK::Chrimson) was generated via Gibson Assembly based on pNH12 (*pmyo*-2::MacQ::mCitrine) and pAAV-hSyn-BiPOLES-mCerulean, using the restriction enzymes BamHI and HindIII and primers pmyo2Tandem_fwd (5'-ggacgaggacacagtgtgaaAAGAGCAGGATCACCAGC-3') [SEQ ID NO. 11] and pmyo2Tandem_rev (5'-agacgacctgtgatgccatgCCCCGAGGGTTAAAATGAAAAG-3') [SEQ ID NO. 12].
pAB31 (*punc*-47::GtACR2::mCerulean::bHK::Chrimson) was generated via Gibson Assembly based on pAB28 (*punc*-47::QuasAr::GFP) and pAAV-hSyn-BiPOLES-mCerulean, using the restriction enzymes AgeI and EcoRI and primers punc-47_BIPOLES_fwd (5'-acatttatttcattacagcaATGGCATCACAGGTCGTC-3') [SEQ ID NO. 13] and punc-47_BIPOLES_rev (5'-agcgaccggcgctcagttggTCACACTGTGTCCTCGTC-3') [SEQ ID NO. 14].

**4. OVC voltage imaging experiments**

For all voltage imaging experiments, ATR (Sigma-Aldrich, USA) had to be supplied to the animals. Therefore, one day prior to each experiment, transgenic L4 stage worms were transferred onto NGM plates, seeded with OP50 bacterial suspension supplemented with ATR (Note: To avoid the interfering fluorescent signal of unbound retinal, the ATR concentration was adjusted for each tissue tested. Final ATR concentration: BWMs: 0.01 mM, Pharynx: 0.03 mM, cholinergic neurons: 0.1 mM, GABAergic neurons: 0.005 mM). For imaging, animals were immobilized with polystyrene beads (0.1 $\mu$m diameter, at 2.5% w/v, Sigma-Aldrich) on top of 10 % agarose pads (in M9 buffer). Voltage-dependent fluorescence of QuasAr was excited with a 637 nm red laser (OBIS FP 637LX, Coherent) at 1.8 W/mm$^2$ and imaged at 700 nm (700/75 ET Bandpass filter, integrated in Cy5 filter cube, AHF Analysentechnik), while optogenetic actuators (BiPOLES, ACR2 or ChR2(H134R)) were controlled by a monochromator (Polychrome V, formerly Till Photonics now Thermo Scientific, purchasable on customer demand), ranging from 400 to 600 nm at 300 $\mu$W/mm$^2$. Imaging was performed on an inverted microscope (Zeiss Axio Observer Z1), equipped with a 40x oil immersion objective (Zeiss EC Plan-NEOFLUAR 40x/ N.A. 1.3, Oil DIC $\infty$ / 0.17), a laser beam splitter (HC BS R594 lambda/2 PV flat, AHF Analysentechnik), a galilean beam expander (BE02-05-A, Thorlabs) and an EMCCD Camera (Evolve 512 Delta, Photometrics). All OVC experiments were performed at $\approx$ 90 fps with 10 ms exposure and a binning of 4x4 (computer specifications: 24 GB RAM, AMD FX(tm)-8150 Eight-Core processor (3.6 GHz), NVIDIA GeForce GT 520). To induce pumping under imaging conditions, animals were incubated in 3 $\mu$l serotonin (20 mM, in M9 buffer) for 3 min prior to the experiments.

**5. OVC software**

The OVC control software was written in Beanshell, the scripting language used by the open source microscopy software Micro-Manager (Edelstein *et al.,* 2010).
Experiments were initiated via the build-in Micro-Manager script panel. All scripts provided by this study run on Micro-Manager v. 1.4.22. For support of Polychrome V related commands, a copy of the Java archive file *TILLPolychrome.jar* must be placed into the MMPlugins folder and the dynamic link libary file *TILLPolychromeJ.dll* into the Sys32 folder (both files were provided by Till Photonics). The OVC software is compatible to all cameras that are supported by Micro-Manager. Before running the software, rectangular ROIs must be selected in the live image mode by using the ROI button that is integrated in the Micro-Manager main window. Once a ROI is selected, the script can be executed via the script panel GUI. An input tab opens, prompting the user to select the OVC- and acquisition parameters. The standard software allows to define a holding $\Delta F/F_0$ value (in %) and the number of frames for each of the steps (3-step-protocol). Moreover, one can select the number of frames for the calibration period, the tolerance range (in %), the start sign of the increment factor that decides, whether to in- or decrease the wavelength with respect to the current $\Delta F/F_0$, the starting wavelength and the wavelength limits (in nm). The free selectability of these parameters ensures that the program can also be used for other/future combinations of optogenetic tools with different spectral properties. For acquisition, light intensity of the Polychrome V (in %), exposure time of the camera (in ms) and camera binning (1x1, 2x2, 4x4 or 8x8) can be selected.

During calibration, incoming grey values are stored in an array and used to evaluate the parameters for the exponential decay function to correct for bleaching of the voltage sensor live during the clamping phase (Equation (1) to (3) below, Miura *et al.,* 2014)). At the last time point of the calibration phase, the first 50 bleaching corrected gray values of the recording are used to calculate Fo. Subsequently, bleaching corrected $\Delta F/F_0$ values are calculated at each time point of the clamping phase (Equation 4).

$$\text{exp. offset} = a \times e^{-b \times frame} + c \qquad (1)$$

$$\text{ratio} = \text{exp. offset}_0 \div \text{exp. offset}_{frame} \qquad (2)$$

$$Fc_{orrect} = F_{frame} \times \text{ratio} \quad (3)$$

$$\Delta F/F_{0\ correct} = ((F_{correct} - F_0) / F_0) * 100 \quad (4)$$

$$multiplier = |\Delta F/F_{0\ holding} - \Delta F/F_0| \quad (5)$$

$$wavelength = wavelength_{frame-1} + multiplier * increment \quad (6)$$

exp. offset: Exponential offset. a, b, c: Exponential fitting parameters. ratio: Ratio between first and current exponential offset. $F_{correct}$: Bleaching-corrected gray values. $F_{frame}$: Gray value of current frame. $\Delta F/F_0$ *Correct:* Bleaching-corrected relative change in fluorescence. Fo: Resting fluorescence value. Equations 1-3 based on the ImageJ Plugin "Correctbleach" (Miura *et al.,* 2014).

During the clamping phase, the current bleaching corrected $\Delta F/F_0$ value is compared to the selected holding value, by calculating the deviation between command and current relative change in fluorescence. If the tolerance range is exceeded, the software reacts with a wavelength change of the monochromator, depending on both the strength, as well as the sign of the deviation (Equation (5) and (6)). When the OVC acquisition is complete, a results text file is output, and the respective image stack is saved automatically as TIFF file. The results file comprises the selected parameters, the bleaching correction function and its quality and the framerate, as well as time, (bleaching corrected-) raw grey- and $\Delta F/F_0$ values, and wavelength traces. In addition to that, status information (system on hold, adapting or wavelength limits reached) is recorded for each time point of the clamp phase.

For the "on-the-run" mode, an additional control window opens as soon as the measurement starts. Here, the holding $\Delta F/F_0$ values can be selected live, either via a range slider or with help of the arrow keys of the keyboard, while a live status update is displayed.

### 6. Electrophysiology in *C. elegans*

Electrophysiological recordings from body wall muscle cells were conducted in immobilized and dissected adult worms as described previously (Liewald *et al.,* 2008). Animals were immobilized with Histoacryl glue (B. Braun Surgical, Spain) and a lateral incision was made to access neuromuscular junctions (NMJs) along the anterior ventral nerve cord. The basement membrane overlying body wall muscles was enzymatically removed by incubation in 0.5 mg/ml collagenase for 10 s (C5138, Sigma-Aldrich, Germany). Integrity of body wall muscle cells and nerve cord was visually examined via DIC microscopy. Recordings from body wall muscles were acquired in whole-cell patch-clamp mode at room temperature (20-22°C) using an EPC-10 amplifier equipped with Patchmaster software (HEKA, Germany).

The head stage was connected to a standard HEKA pipette holder for fire-polished borosilicate pipettes (1B100F-4, Worcester Polytechnic Institute, Worcester, MA, USA) of 4-10 MΩ resistance. The extracellular bath solution consisted of 150 mM NaCl, 5 mM KCl, 5 mM $CaCl_2$, 1 mM $MgCl_2$, 10 mM glucose, 5 mM sucrose, and 15 mM HEPES (pH 7.3 with NaOH, ≈330 mOsm). The internal/patch pipette solution consisted of K-gluconate 115 mM, KCl 25 mM, $CaCl_2$ 0.1 mM, $MgCl_2$ 5 mM, BAPTA 1 mM, HEPES 10 mM, $Na_2ATP$ 5 mM, $Na_2GTP$ 0.5 mM, cAMP 0.5 mM, and cGMP 0.5 mM (pH 7.2 with KOH, ≈320 mOsm).

For light activation a monochromator (Polychrome V, Thermo Scientific) was used, ranging from 400 to 600 nm at 300 μW/mm$^2$. To create conditions as similar as possible to those for the OVC experiments, additional excitation was performed with a laser at 637 nm. Subsequent analysis and graphing were performed using Patchmaster, and Origin (Originlabs).

Electrophysiological recordings from DVB were conducted in immobilized and dissected adult worms as described previously (Husson *et al.,* 2012). with minor modifications. Both dissection and recording were performed at room temperature. Briefly, an animal was immobilized with cyanoacrylate adhesive (Vetbond tissue adhesive; 3M) along the ventral side of the posterior body on a Sylgard 184-coated (Dow Corning) glass coverslip. A small longitudinal incision was made using a diamond dissecting blade (Type M-DL 72029 L; EMS) in the tail region along the glue line. The cuticle flap was folded back and glued to the coverslip with GLUture Topical Adhesive (Abbott Laboratories), exposing DVB. The coverslip with the dissected preparation was then placed into a custom-made open recording chamber (~1.5 ml volume) and treated with 1 mg/ml collagenase (type IV; Sigma) for -10 s by hand pipetting. The recording chamber was subsequently perfused with the standard extracellular solution using a custom-made gravity-feed perfusion system for -10 ml. The standard pipette solution was (all concentrations in mM): [K-gluconate 115; KCl 15; KOH 10; MgCl2 5; CaCl2 0.1; Na2ATP 5; NaGTP 0.5; Na-cGMP 0.5; cAMP 0.5; BAPTA 1; Hepes 10; Sucrose 50], with pH adjusted with KOH to 7.2, osmolarity 320-330 mOsm. The standard extracellular solution was: [NaCl 140; NaOH 5; KCL 5; CaCl2 2; MgCl2 5; Sucrose 15; Hepes 15; Dextrose 25], with pH adjusted with NaOH to 7.3, osmolarity 330-340 mOsm. Liquid junction potentials were calculated and corrected before recording. Electrodes with resistance (RE) of 20-30 MΩ were made from borosilicate glass pipettes (BF100-58-10; Sutter Instruments) using a laser pipette puller (P-2000; Sutter Instruments) and fire-polished with a microforge (MF-830; Narishige). Recordings were performed using single-electrode whole-cell current clamp (Heka, EPC-10 USB) with two-stage capacitive compensation optimized at rest, and series resistance compensated to 50%.

The features disclosed in the foregoing description, in the claims and/or in the accompanying drawings may, both separately and in any combination thereof, be material for realizing the invention in diverse forms thereof.

REFERENCES

Akerboom, J. et al. Genetically encoded calcium indicators for multi-color neural activity imaging and combination with optogenetics. Frontiers in molecular neuroscience 6, 2; 10.3389/fnmol.2013.00002. (2013).

Avery, L., and Thomas, J.H. (1997). Feeding and Defecation, In C. elegans II, D.L. Riddle, T. Blumenthal, B.J. Meyer, and J.R. Priess, eds. (Cold Spring Harbor Laboratory Press), pp. 679-716.

Bergs, A. et al. Rhodopsin optogenetic toolbox v2.0 for light-sensitive excitation and inhibition in Caenorhabditis elegans. PloS one 13, e0191802; 10.1371/journal.pone.0191802 (2018).

Berndt, A., Yizhar, O., Gunaydin, L. A., Hegemann, P. & Deisseroth, K. Bi-stable neural state switches. Nature neuroscience 12, 229-234; 10.1038/nn.2247 (2009).

Chen, T.-W. et al. Ultrasensitive fluorescent proteins for imaging neuronal activity. Nature 499, 295-300; 10.1038/nature12354 (2013).

Dawydow, A. et al. Channelrhodopsin-2-XXL, a powerful optogenetic tool for low-light applications. Proceedings of the National Academy of Sciences of the United States of America 111, 13972-13977; 10.1073/pnas.1408269111 (2014).

Edelstein, A., Amodaj, N., Hoover, K., Vale, R. & Stuurman, N. Computer control of microscopes using μManager. Current protocols in molecular biology Chapter 14, Unit14.20; 10.1002/0471142727.mb1420s92. (2010).

Evans, T. Transformation and microinjection. WormBook; 10.1895/wormbook.1.108.1 (2006).

Fan, L. Z. et al. All-Optical Electrophysiology Reveals the Role of Lateral Inhibition in Sensory Processing in Cortical Layer 1. Cell 180, 521-535.e18; 10.1016/j.cell.2020.01.001 (2020).

Francis, M. M., Mellem, J. E. & Maricq, A. V. Bridging the gap between genes and behavior: recent advances in the electrophysiological analysis of neural function in Caenorhabditis elegans. Trends in Neurosciences 26, 90-99; 10.1016/S0166-2236(02)00041-3 (2003).

Gong, Y., Li, J. Z. & Schnitzer, M. J. Enhanced Archaerhodopsin Fluorescent Protein Voltage Indicators. PloS one 8, e66959; 10.1371/journal.pone.0066959 (2013).

Gong, Y., Wagner, M. J., Zhong Li, J. & Schnitzer, M. J. Imaging neural spiking in brain tissue using FRET-opsin protein voltage sensors. Nature communications 5, 3674; 10.1038/ncomms4674 (2014).

Govorunova, E. G., Sineshchekov, O. A., Janz, R., Liu, X. & Spudich, J. L. NEUROSCIENCE. Natural light-gated anion channels: A family of microbial rhodopsins for advanced optogenetics. Science (New York, N.Y.) 349, 647-650; 10.1126/science.aaa7484 (2015).

Azimi Hashemi, N. et al. Rhodopsin-based voltage imaging tools for use in muscles and neurons of Caenorhabditis elegans. Proceedings of the National Academy of Sciences of the United States of America 116, 17051-17060; 10.1073/pnas.1902443116 (2019).

Hochbaum, D. R. et al. All-optical electrophysiology in mammalian neurons using engineered microbial rhodopsins. Nature methods 11, 825-833; 10.1038/nmeth.3000 (2014).

Husson, S. J. et al. Microbial light-activatable proton pumps as neuronal inhibitors to functionally dissect neuronal networks in C. elegans. PloS one 7, e40937; 10.1371/journal.pone.0040937 (2012).

Kralj, J. M., Douglass, A. D., Hochbaum, D. R., Maclaurin, D. & Cohen, A. E. Optical recording of action potentials in mammalian neurons using a microbial rhodopsin. Nature methods 9, 90-95; 10.1038/nmeth.1782 (2011).

Liewald, J. F. et al. Optogenetic analysis of synaptic function. Nature methods 5, 895-902; 10.1038/nmeth.1252 (2008).

Lin, M. Z. & Schnitzer, M. J. Genetically encoded indicators of neuronal activity. Nature neuroscience 19, 1142-1153; 10.1038/nn.4359 (2016).

McIntire, S. L., Jorgensen, E., Kaplan, J. & Horvitz, H. R. The GABAergic nervous system of Caenorhabditis elegans. Nature 364, 337-341; 10.1038/364337a0 (1993).

Miura, K., Rueden, C., Hiner, M., Schindelin, J. & Rietdorf, J. Imagej Plugin Correctbleach V2.0.2 (Zenodo, 2014).

Newman, J. P. et al. Optogenetic feedback control of neural activity. eLife 4, e07192; 10.7554/eLife.07192 (2015).
Piatkevich, K. D. et al. A robotic multidimensional directed evolution approach applied to fluorescent voltage reporters. Nature chemical biology 14, 352-360; 10.1038/s41589-018-0004-9 (2018)
Richmond, J. E., Davis, W. S. & Jorgensen, E. M. UNC-13 is required for synaptic vesicle fusion in C. elegans. Nature neuroscience 2, 959-964; 10.1038/14755 (1999).
Schultheis, C., Liewald, J. F., Bamberg, E., Nagel, G. & Gottschalk, A. Optogenetic long-term manipulation of behavior and animal development. PloS one 6, e18766; 10.1371/journal.pone.0018766 (2011).
Vierock, J. et al. BiPOLES: a tool for bidirectional dual-color optogenetic control of neurons. bioRxiv 2020.07.15.204347; doi: https://doi.org/10.1101/2020.07.15.204347.
Wang, H. & Sieburth, D. PKA controls calcium influx into motor neurons during a rhythmic behavior. PLoS genetics 9, e1003831; 10.1371/journal.pgen.1003831 (2013).
Zhang F, Wang LP, Brauner M, Liewald JF, Kay K, Watzke N, Wood PG, Bamberg E, Nagel G, Gottschalk A, Deisseroth K. Multimodal fast optical interrogation of neural circuitry. Nature. 2007 Apr 5;446(7136):633-9. doi: 10.1038/nature05744.

## Claims

1. Use of a genetically encoded voltage indicator (GEVI) protein and counteracting optogenetic protein(s) for depolarization and hyperpolarization as an optical voltage clamp in a cell and/or for optical control of the membrane potential in a cell.

2. The use of claim 1, wherein the cell is an electrically excitable cell, such as a muscle cell, a neuron, a cardiomyocyte (such as iPSC-derived cardiomyocyte) or a glial cell, or wherein the cell is a cell in which membrane potential is subject to dynamic changes or where changes in membrane potential affect membrane transport processes.

**3.** The use of claim 1 or 2, wherein the GEVI protein is

- an opsin-based GEVI protein

  such as rhodopsin-based, e.g. Arch, QuasAr, Archon, or
  eFRET-based or FP-retinal FRET, e.g. Ace-mNeon, VARNAM, macQ-mCitrine, QuasAr2-mOrange2, paQuasAr3

or
- a voltage-sensing domain-based GEVI protein

  such as FP-FP FRET, e.g. Mermaid, VSFP Butterflies, or
  monochromatic FP, e.g. FlicR, ArcLight, ASAP;

and/or wherein the counteracting optogenetic protein(s) for depolarization and hyperpolarization are

(i) a pair of optogenetic proteins, wherein one is for depolarization and the other is for hyperpolarization, wherein

- the depolarizing optogenetic proteins are:

  - channelrhodopsins (ChRs), such as ChR1, ChR2, VChR1, ChRmine,
  - ChR2 variants or chimeras, such as Chrimson, ReaChR, CheRiff, ChETA, bReaChEs,

- the hyperpolarizing optogenetic proteins are:

  - halorhodopsins, such as eNpHR2.0 and eNpHR3.0,
  - Archaerhodopsins,
  - fungal opsins, such as Mac,
  - bacteriorhodopsins, such as eBR,
  - anion-conducting channelrhodopsins, such as iC1C2, *Gt*ACR1, *Gt*ACR2, Phobos, Aurora, (i-)ChloCs.

(ii) a bidirectional optogentic protein,
such as

- a fusion protein of blue-light-sensitive anion-conducting channelrhodopsin *Gt*ACR2 fused to the red-light-sensitive cation-conducting channelrhodopsin Chrimson (BiPOLES),
- *Gt*ACR2 fused to bReaChEs,
- *Gt*ACR2 fused to ChRmine,
- fusion proteins of any other depolarizing and hyperpolarizing rhodopsins, as defined in (i), whose action spectral peaks are sufficiently separated.

**4.** The use of any one of claims 1 to 3, comprising
studying muscular ensembles or neuronal networks,
studying animal models, which are preferably transparent,
studying or providing models of neurodegenerative diseases,
studying or providing models of cardiac diseases,
functional characterization of ion channels,
expression analysis screening of ion channels, and/or
screening for drugs that affect ion channels,
wherein the ion channels can be ryanodine receptor, sodium channels, calcium channels, potassium channels (such as HERG K+ channel), ligand-gated ion channels,
wherein the screening is preferably high-throughput screening.

**5.** A cell co-expressing
a genetically encoded voltage indicator (GEVI) protein, and

counteracting optogenetic protein(s) for depolarization and hyperpolarization.

6. The cell of claim 5, which is
an electrically excitable cell, such as a muscle cell, a neuron, a cardiomyocyte (such as iPSC-derived cardiomyocyte) or a glial cell, or
a cell in which membrane potential is subject to dynamic changes or where changes in membrane potential affect membrane transport processes;
wherein the GEVI protein is as defined in claim 3,
and/or the counteracting optogenetic protein(s) for depolarization and hyperpolarization are as defined in claim 3.

7. Use of the cell according to claim 5 or 6 for
optical control of membrane potential,
studying muscular ensembles or neuronal networks,
studying animal models, which are preferably transparent,
studying or providing models of neurodegenerative diseases,
studying or providing models of cardiac diseases,
functional characterization of ion channels,
expression analysis screening of ion channels, and/or
screening for drugs that affect ion channels,
wherein the ion channels can be ryanodine receptor, sodium channels, calcium channels, potassium channels (such as HERG K+ channel), ligand-gated ion channels,
wherein the screening is preferably high-throughput screening.

8. The use of any one of claims 1 to 4 or 7, wherein cells of a patient with a neurodegenerative disease, epilepsy, a cardiac disease (such as heart rhythm dysfunction), a renal disease, a muscular malfunction based on ion channel mutations, a disease based on aberrant mitochondrial membrane potential and/or a disease based on aberrant organellar membrane potential are used.

9. A method for controlling the membrane voltage of a target cell, the method comprising:

(1) incorporating into a target cell, which is preferably a cell as defined in claim 2, a voltage indicator and counteracting optogenetic protein(s) for depolarization and hyperpolarization;
or providing a cell according to any one of claims 6 to 8 as target cell,
(2) exciting the fluorescence of the voltage indicator with a first light source, preferably a laser, an LED or an HBO lamp,
(3) recording the intensity of fluorescence with a camera or a photomultiplier in real time and determining the relative change of fluorescence ($\Delta F/F_0$),
(4) at the same time as (2) and (3), irradiating the counteracting optogenetic protein(s) for depolarization and hyperpolarization with a second light source,
preferably a tunable light source which can access a broad spectrum of wavelengths, but produces a narrow range of any of those wavelengths at a given time, such as a monochromator, a tunable laser,
comprising the adjustment of a feedback wavelength of said second light source depending on (i) the difference between intended membrane voltage and present membrane voltage, reflected by relative changes in fluorescence ($\Delta F/F_0$),and (ii) the absorption maxima of the optogenetic protein(s);
wherein said feedback wavelength is adjusted

(a) towards the absorption maximum of the depolarizing tool, if the present membrane voltage is smaller than a target membrane voltage, whereas the membrane voltage is indicated by the relative change in fluorescence ($\Delta F/F_0$),and
(b) towards the absorption maximum of the hyperpolarizing tool, if the present membrane value is greater than a target membrane voltage, whereas the membrane voltage is indicated by the relative change in fluorescence ($\Delta F/F_0$),

to clamp the membrane potential at the target voltage, reflected by the target $\Delta F/F_0$ value,
which results in an optical closed-loop controlling of the membrane potential of the target cell.

10. A method for determining the functionality of and/or for modulating ion channels and/or for determining influences on ion channels,

the method comprising:

(1) providing a cell, which is preferably a cell as defined in claim 2, comprising the ion channel to be determined for functionality or modulated, and incorporating into said cell a voltage indicator, and counteracting optogenetic protein(s) for depolarization and hyperpolarization;
or providing a cell according to any one of claims 6 to 8 as target cell and introducing the ion channel to be determined for functionality or modulated,
comprising using ion channel variant(s) comprising mutation(s) and/or adding compound(s) to be tested for an effect on the ion channel or its variant(s),
(2) exciting the fluorescence of the voltage indicator with a first light source, preferably a laser, an LED or an HBO lamp,
(3) recording the intensity of fluorescence with a camera or a photomultiplier in real time and determining the relative change of fluorescence ($\Delta F/F_0$),
(4) at the same time as (2) and (3), irradiating the counteracting optogenetic protein(s) for depolarization and hyperpolarization with a second light source,
preferably a tunable light source which can access a broad spectrum of wavelengths, but produces a narrow range of any of those wavelengths at a given time, such as a monochromator, a tunable laser,
comprising the adjustment of a feedback wavelength of said second light source depending on (i) the difference between intended membrane voltage and present membrane voltage, reflected by relative changes in fluorescence ($\Delta F/F_0$),and (ii) the absorption maxima of the optogenetic protein(s);
wherein said feedback wavelength is adjusted

(a) towards the absorption maximum of the depolarizing tool, if the present membrane voltage is smaller than a target membrane voltage, whereas the membrane voltage is indicated by the relative change in fluorescence ($\Delta F/F_0$),and
(b) towards the absorption maximum of the hyperpolarizing tool, if the present membrane voltage is greater than a target membrane voltage, whereas the membrane voltage is indicated by the relative change in fluorescence ($\Delta F/F_0$),

to clamp the membrane potential at the intended membrane voltage, reflected by the target $\Delta F/F_0$ value, which results in an optical closed-loop, and
(5) determining the feedback light parameters of step (4) and comparing them with the feedback light parameters determined for a control, wherein a difference between said feedback light parameters of step (4) and the control indicates that the mutation(s) of the ion channel and/or the added compound(s) have an effect on the ion channel, wherein the feedback light parameters are selected from the feedback wavelength(s), the course of the feedback wavelength(s) and/or the light intensity, and
wherein the control is the wildtype ion channel and/or no compound(s) are added.

**11.** The method of claim 10, wherein the compound(s) to be tested for an effect on the ion channel or its variant(s) are added during steps (3) and (4);
and/or wherein the ion channels can be ryanodine receptor, sodium channels, calcium channels, potassium channels (such as HERG K+ channel), ligand-gated ion channels.

**12.** The method of any one of claims 9 to 11, wherein the voltage indicator is a genetically encoded voltage indicator (GEVI) protein, preferably as defined in claim 3, or a voltage sensitive dye,
and/or wherein the counteracting optogenetic protein(s) for depolarization and hyperpolarization are as defined in claim 3.

**13.** The method of any one of claims 9 to 12, comprising a calibration and bleaching correction,
which preferably comprises measuring the exponential decrease in fluorescence of the voltage indicator, such as a GEVI protein, and real-time correcting each incoming $\Delta F/F_0$ value accordingly,
and/or comprising a compensatory wavelength to counteract optical crosstalk.

**14.** The method of any one of clams 9 to 13, wherein in step (3)
the camera or photomultiplier records gray values and a software translates them into relative changes of fluorescence ($\Delta F/F_0$).

**15.** The method of any one of clams 11 to 14, furthermore comprising one or more of the following:

(i) selecting a tolerance range for the difference between intended membrane voltage and present membrane voltage, reflected by relative changes in fluorescence ($\Delta F/F_0$),
preferably 0.1 to 10%, more preferably 0.2 to 5%, such as 1%;
(ii) weighting of the feedback light depending on the size of deviation between intended membrane voltage and present membrane voltage, reflected by relative changes in fluorescence ($\Delta F/F_0$),
(iii) adapting the wavelength of the second light source in real time in increments, which depend on the size of deviation between intended membrane voltage and present membrane voltage, reflected by relative changes in fluorescence ($\Delta F/F_0$),as soon as the present $\Delta F/F_0$ value violates a selected tolerance range,
(iv) preselecting the target membrane voltage, as indicated by the relative change in fluorescence ($\Delta F/F_0$), or changing it in real time.

# Figure 1 A

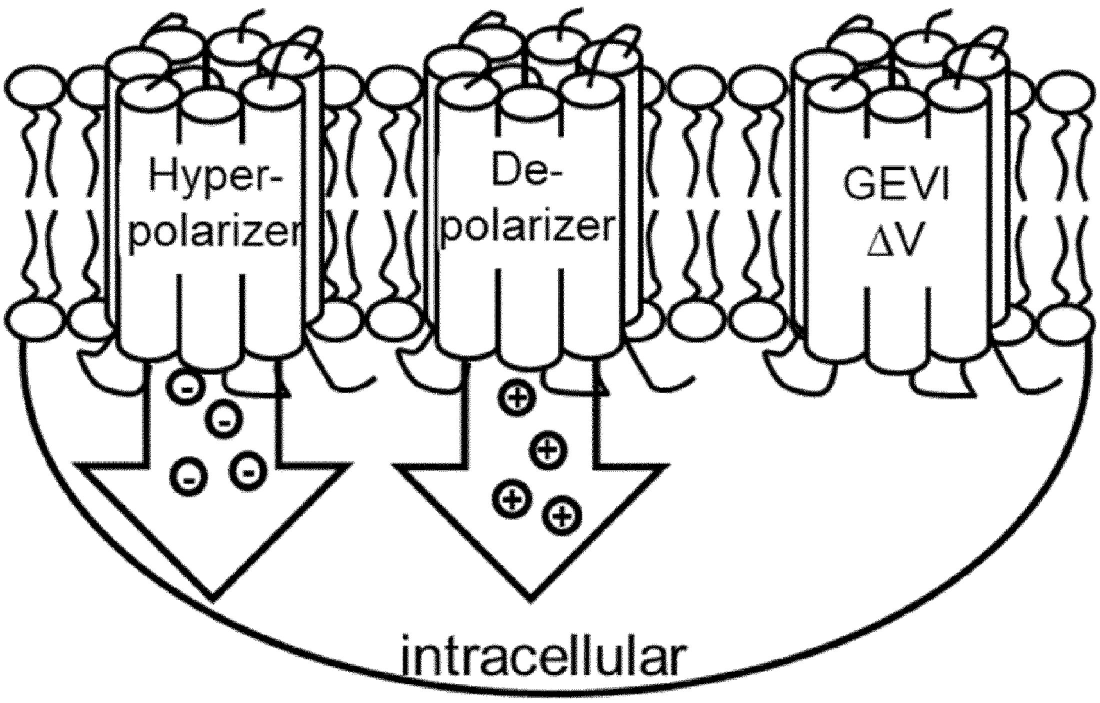

Figure 1 B

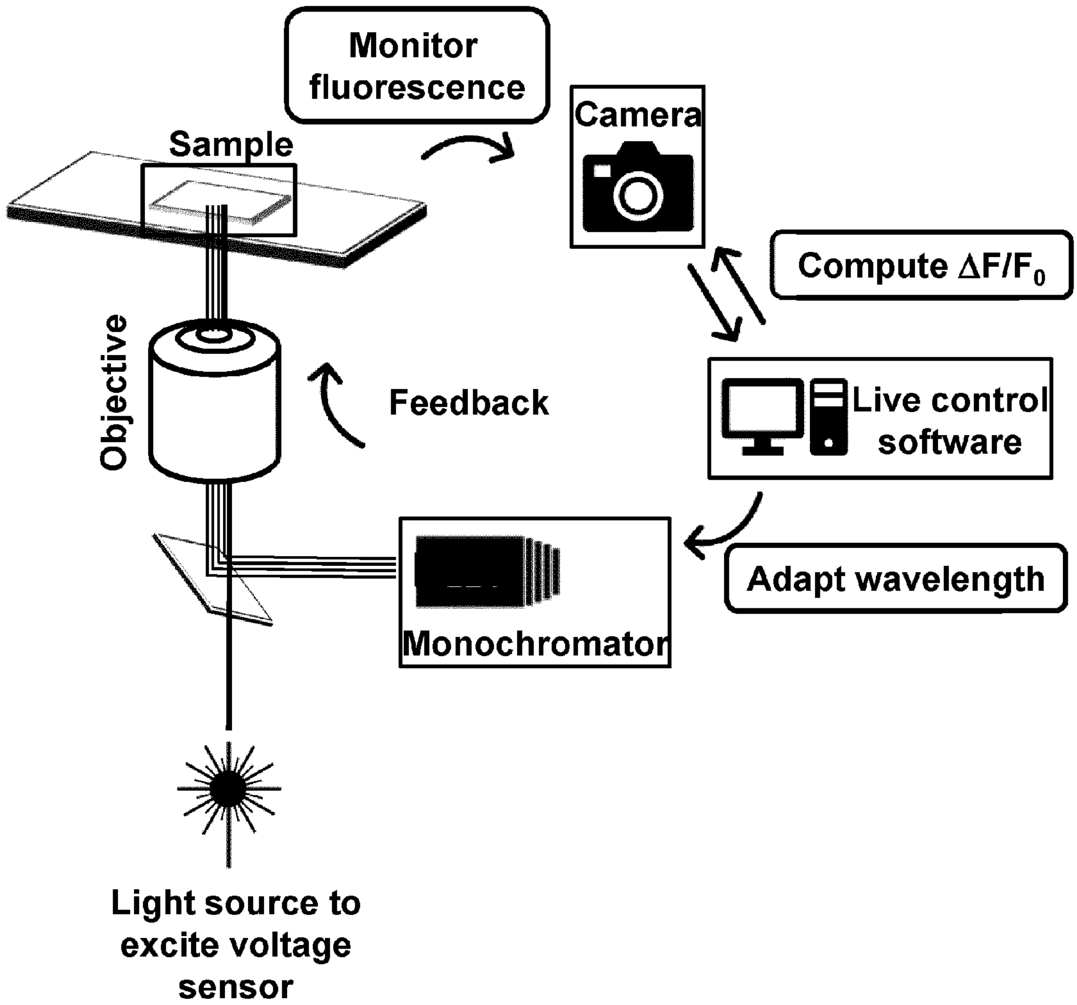
Monitor fluorescence
Camera
Sample
Compute ΔF/F0
Objective
Feedback
Live control software
Adapt wavelength
Monochromator
Light source to excite voltage sensor

# Figure 1 C

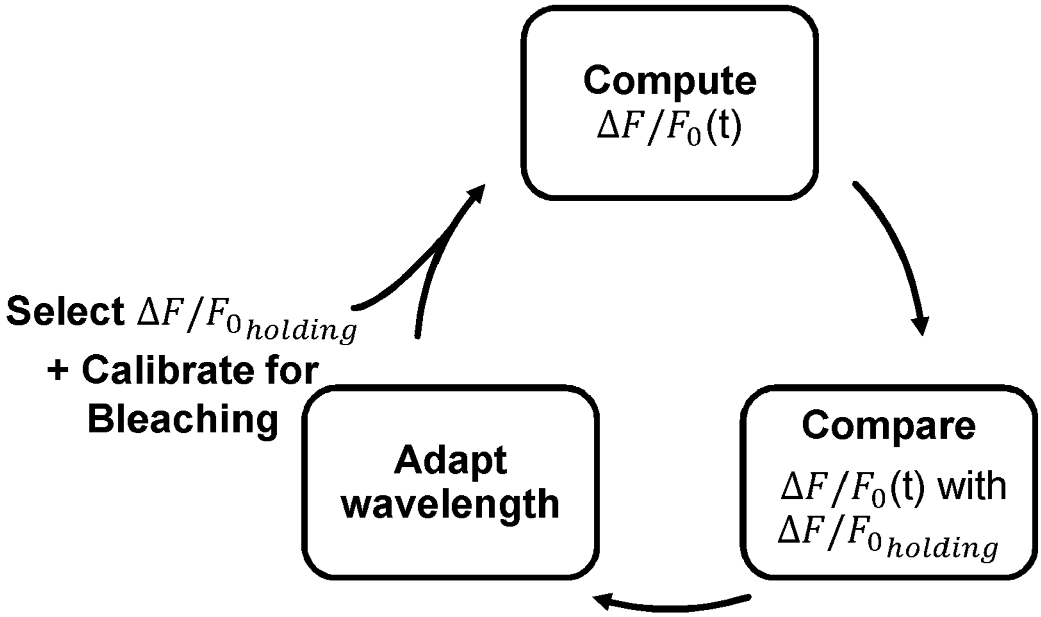
Compute
$\Delta F/F_0$(t)
Compare
$\Delta F/F_0$(t) with
$\Delta F/F_{0_{holding}}$
Adapt
wavelength
Select $\Delta F/F_{0_{holding}}$
+ Calibrate for
Bleaching

Figure 2 A

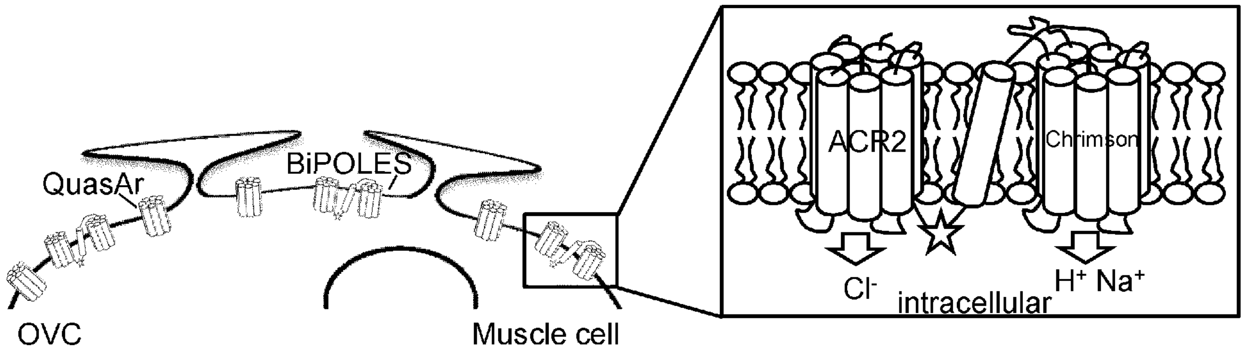
QuasAr
BiPOLES
OVC
Muscle cell
ACR2
Chrimson
Cl-
intracellular
H+ Na+

Figure 2 B

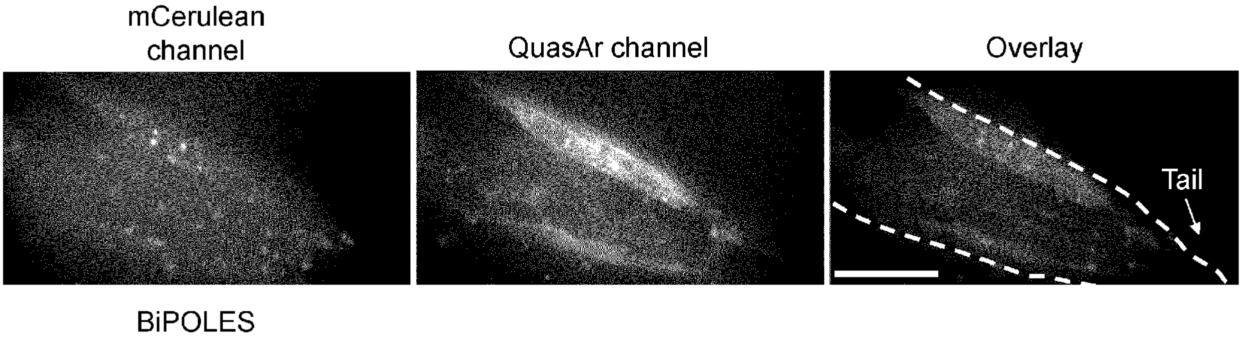

Figure 2 C

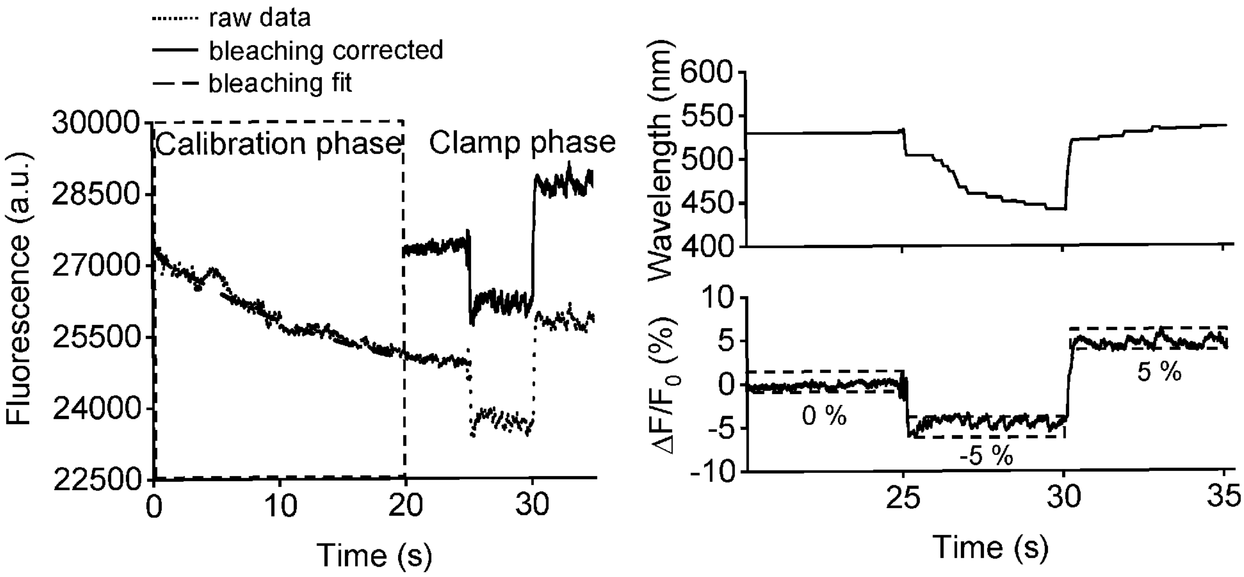
raw data
bleaching corrected
bleaching fit
Calibration phase
Clamp phase
Fluorescence (a.u.)
30000
28500
27000
25500
24000
22500
0
10
20
30
Time (s)
Wavelength (nm)
600
550
500
450
400
ΔF/F0 (%)
10
5
0
-5
-10
0 %
-5 %
5 %
25
30
35
Time (s)

## Figure 2 D

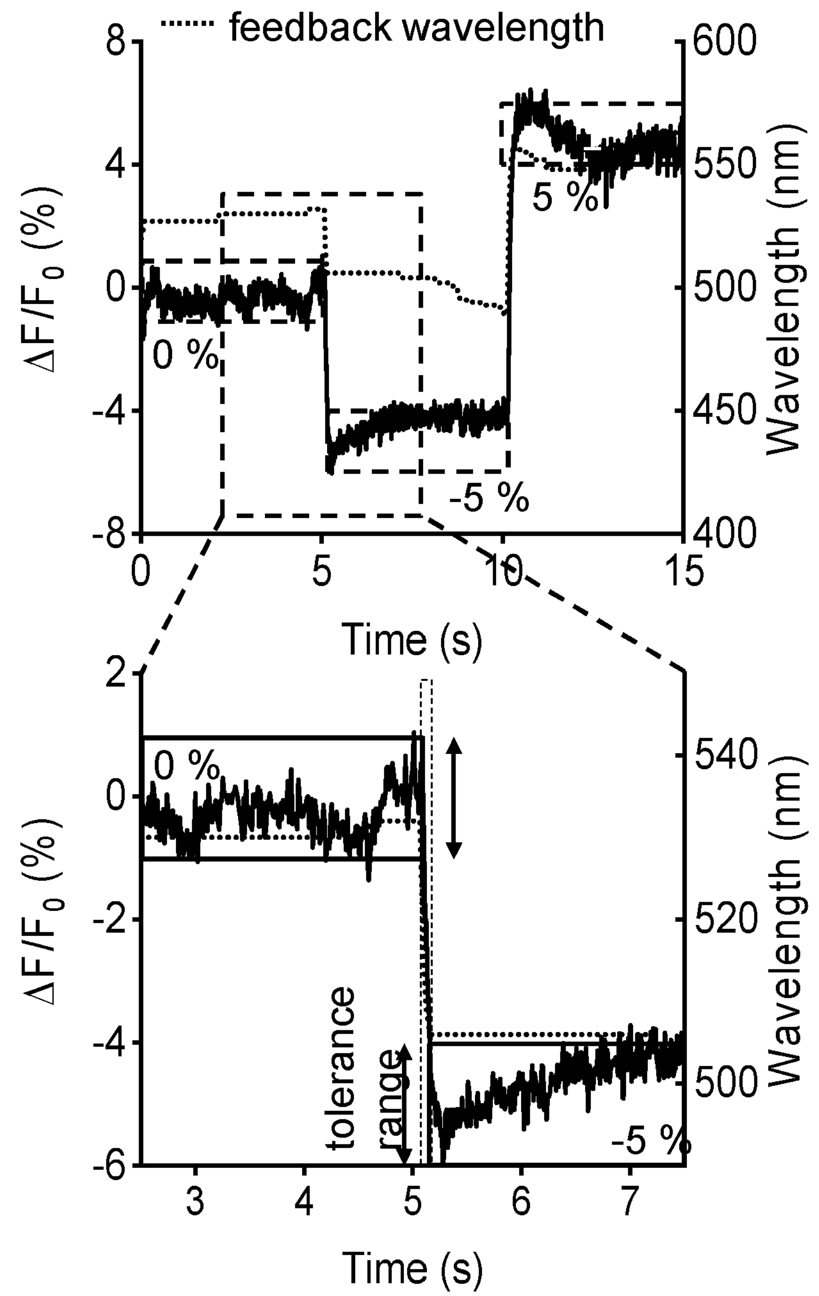
feedback wavelength
8
4
0
-4
-8
ΔF/F0 (%)
0 %
-5 %
5 %
600
550
500
450
400
Wavelength (nm)
0
5
10
15
Time (s)
2
0
-2
-4
-6
ΔF/F0 (%)
0 %
tolerance range
-5 %
540
520
500
Wavelength (nm)
3
4
5
6
7
Time (s)

# Figure 2 E

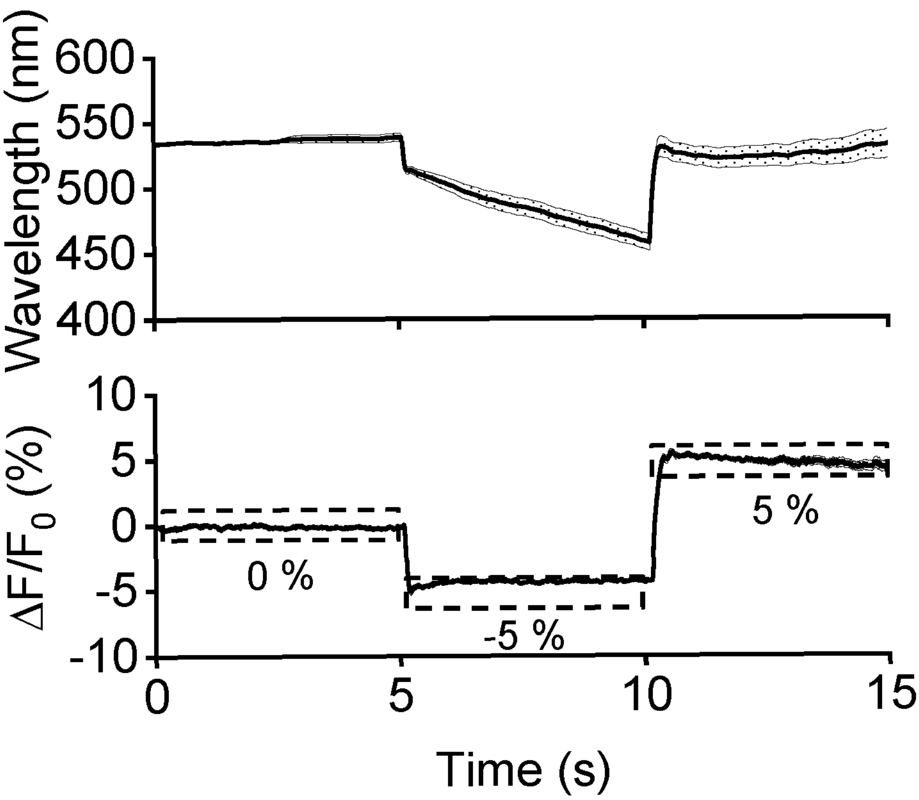
Wavelength (nm)
600
550
500
450
400
ΔF/F0 (%)
10
5
0
-5
-10
0 %
-5 %
5 %
0
5
10
15
Time (s)

## Figure 2 F to H

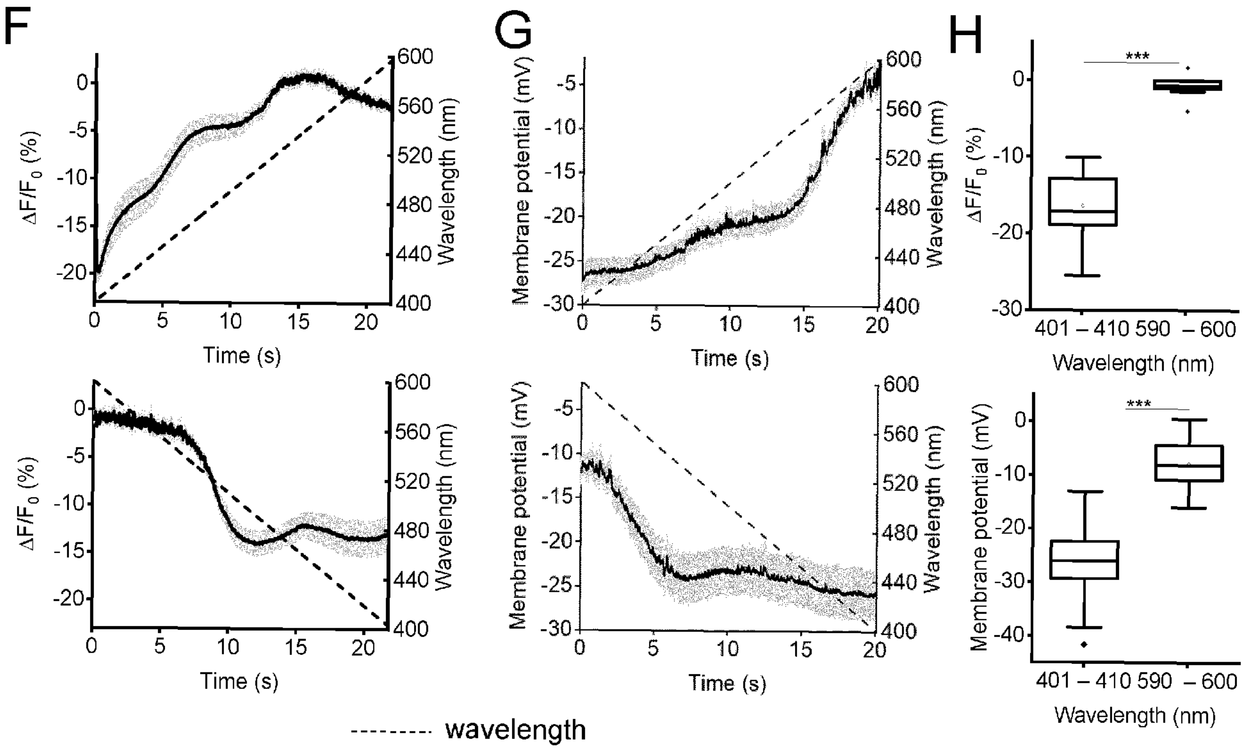

**Figure 2 I**

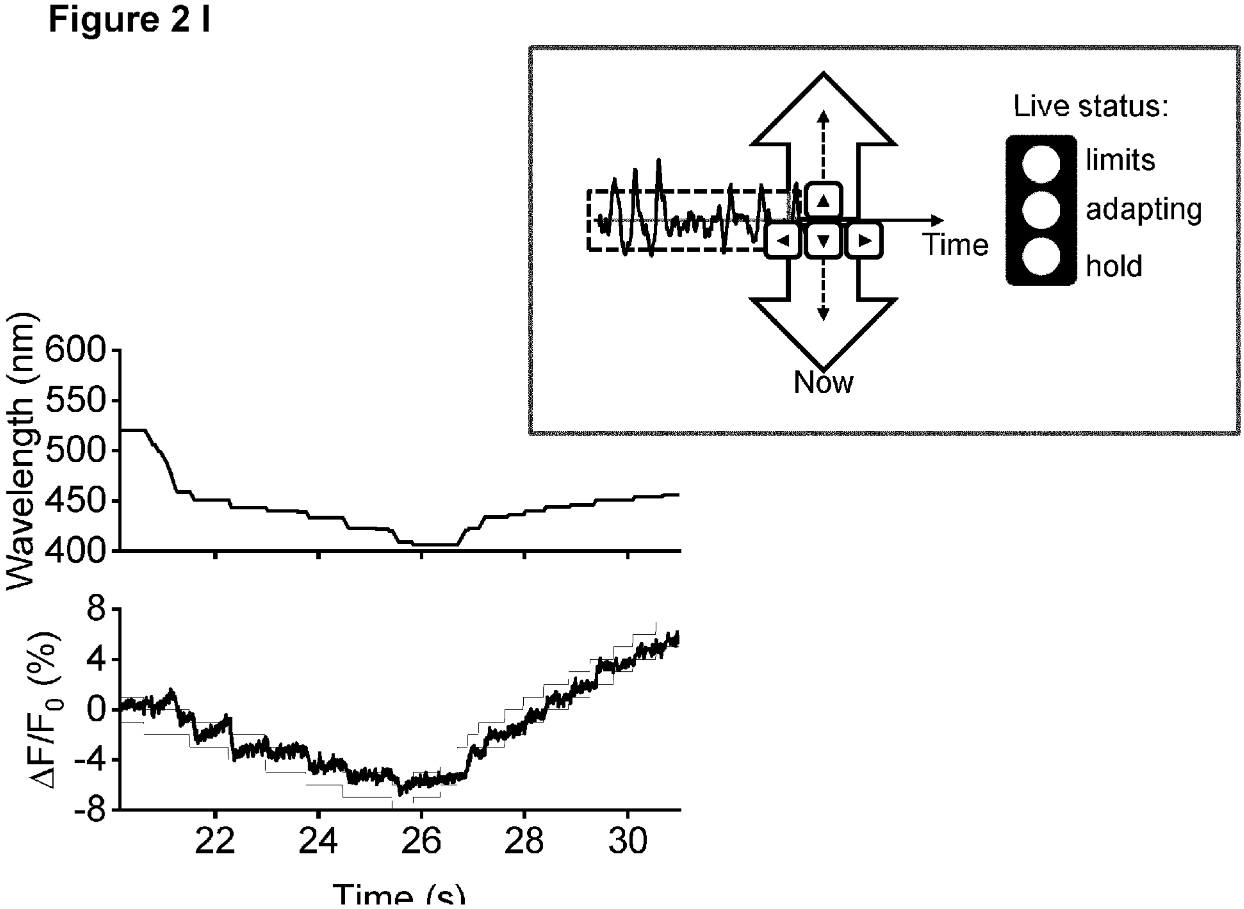
Live status:
limits
adapting
hold
Time
Now
Wavelength (nm)
600
550
500
450
400
ΔF/F0 (%)
8
4
0
-4
-8
22
24
26
28
30
Time (s)

# Figure 3 A

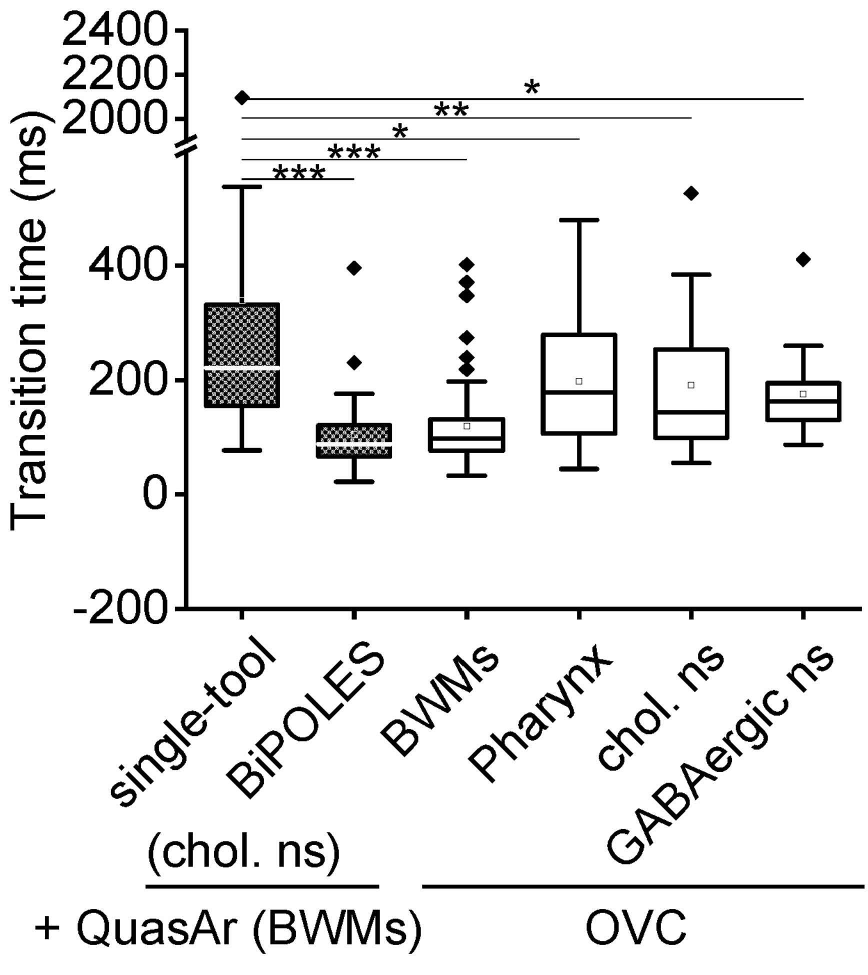
Transition time (ms)
2400
2200
2000
400
200
0
-200
*
**
*
***
***
single-tool
BiPOLES
BWMs
Pharynx
chol. ns
GABAergic ns
(chol. ns)
+ QuasAr (BWMs)
OVC

## Figure 3 B

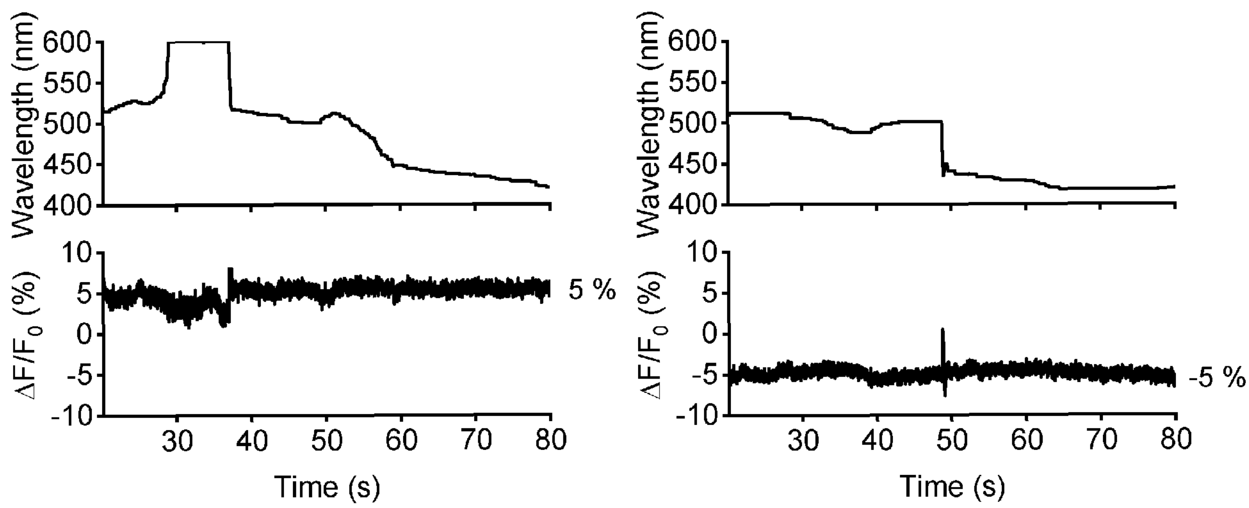
Wavelength (nm)
600
550
500
450
400
ΔF/F0 (%)
10
5
0
-5
-10
5 %
30 40 50 60 70 80
Time (s)
-5 %

Figure 3 C

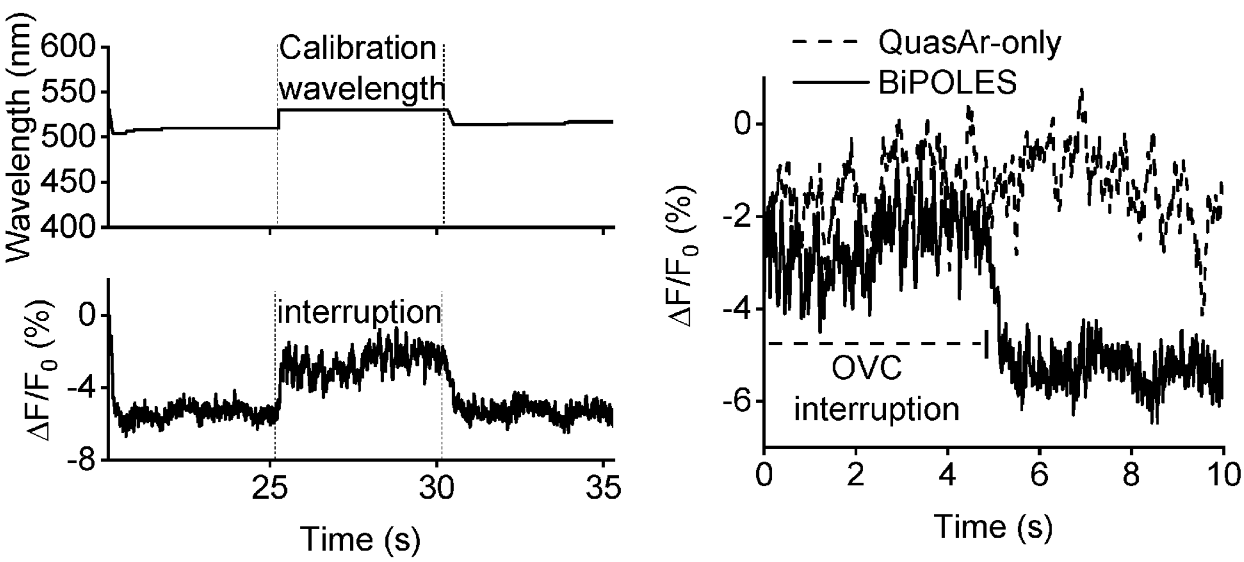
Wavelength (nm)
600
550
500
450
400
Calibration
wavelength
ΔF/F0 (%)
0
-4
-8
interruption
25
30
35
Time (s)
QuasAr-only
BiPOLES
0
-2
-4
-6
OVC
interruption
0
2
4
6
8
10
Time (s)

Figure 3 D

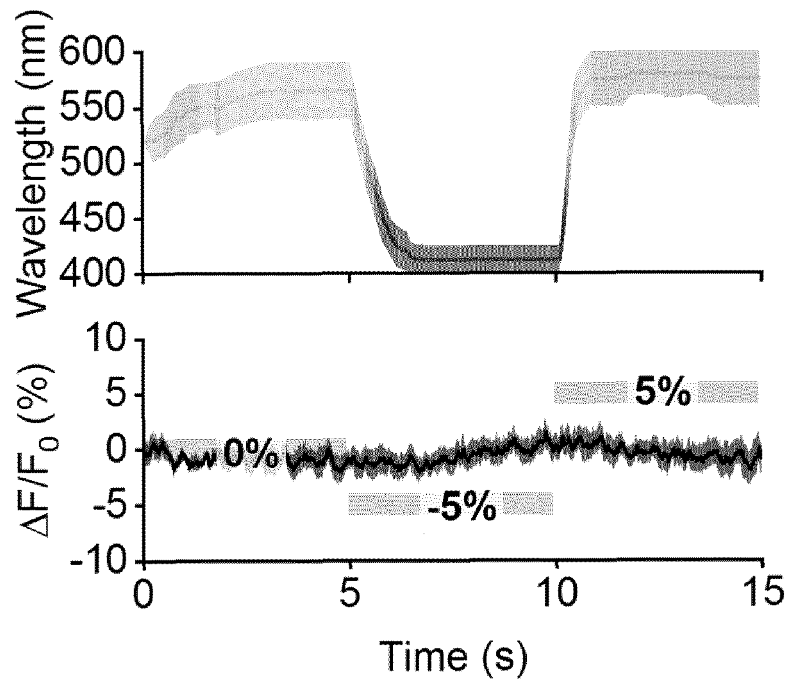
Wavelength (nm)
600
550
500
450
400
ΔF/F0 (%)
10
5
0
-5
-10
0%
5%
-5%
0
5
10
15
Time (s)

Figure 4 A

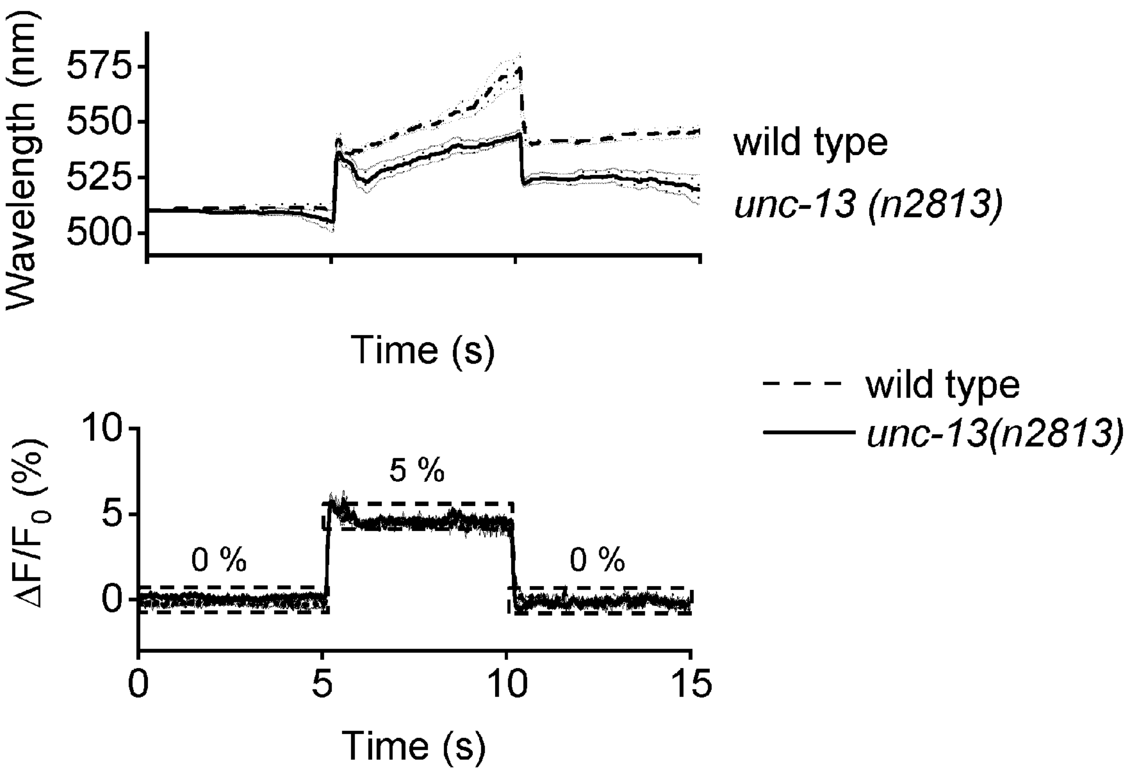
Wavelength (nm)
575
550
525
500
wild type
unc-13 (n2813)
Time (s)
wild type
unc-13(n2813)
ΔF/F0 (%)
10
5
0
5 %
0 %
0 %
0
5
10
15
Time (s)

## Figure 4 B

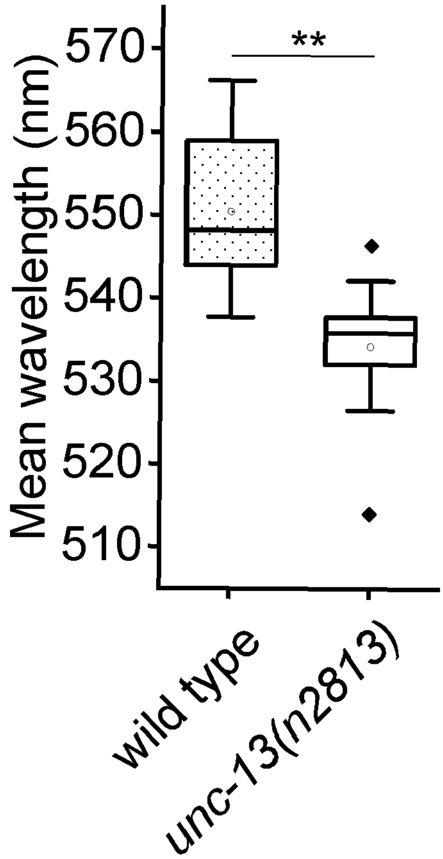
Mean wavelength (nm)
570
560
550
540
530
520
510
**
wild type
unc-13(n2813)

## Figure 5 A

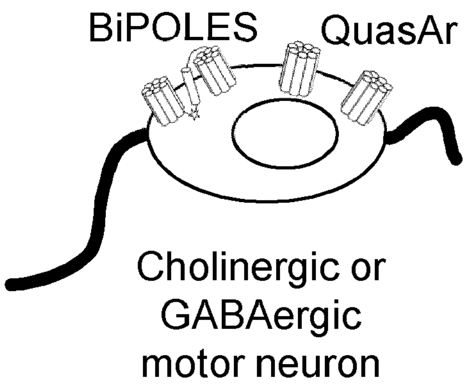

Figure 5 B

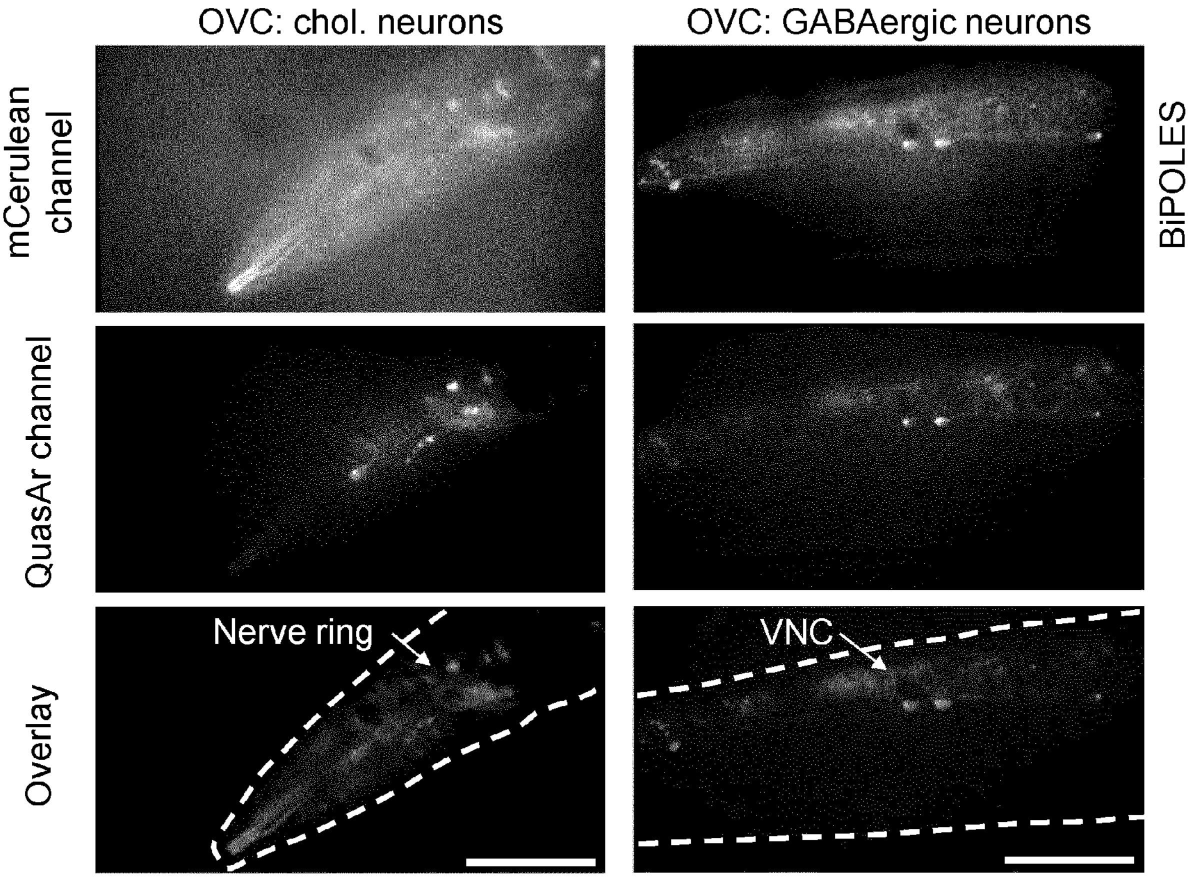
OVC: chol. neurons
OVC: GABAergic neurons
mCerulean channel
QuasAr channel
Overlay
BiPOLES
Nerve ring
VNC

Figure 5 C

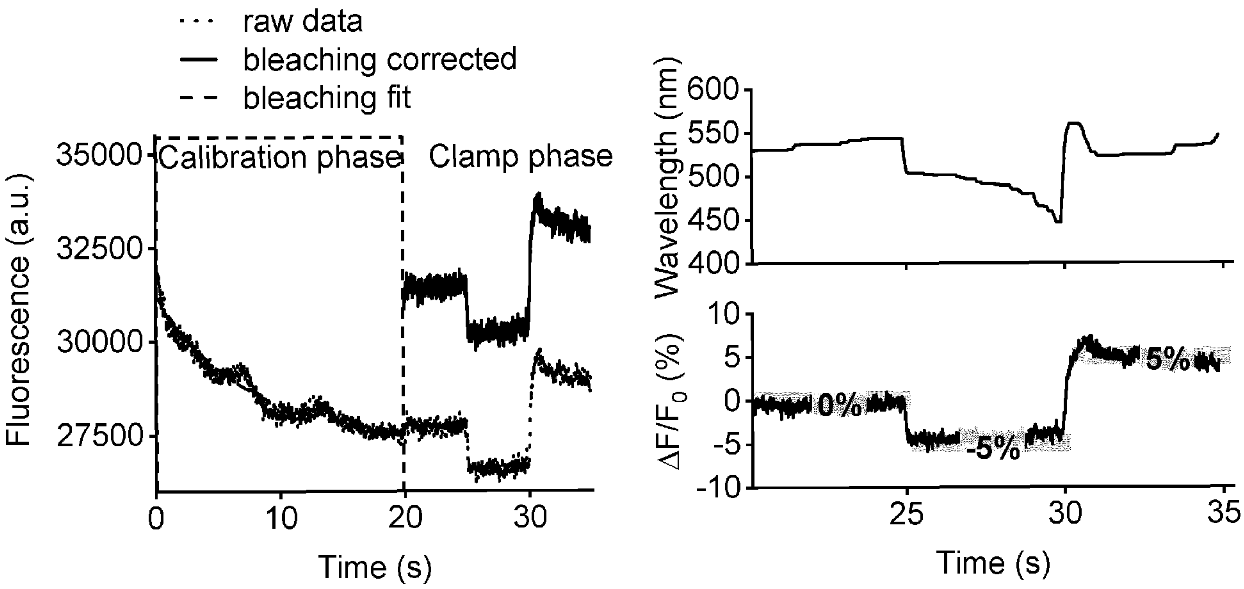
raw data
bleaching corrected
bleaching fit
Calibration phase
Clamp phase
Fluorescence (a.u.)
35000
32500
30000
27500
0
10
20
30
Time (s)
Wavelength (nm)
600
550
500
450
400
ΔF/F0 (%)
10
5
0
-5
-10
0%
-5%
5%
25
30
35
Time (s)

Figure 5 D and E

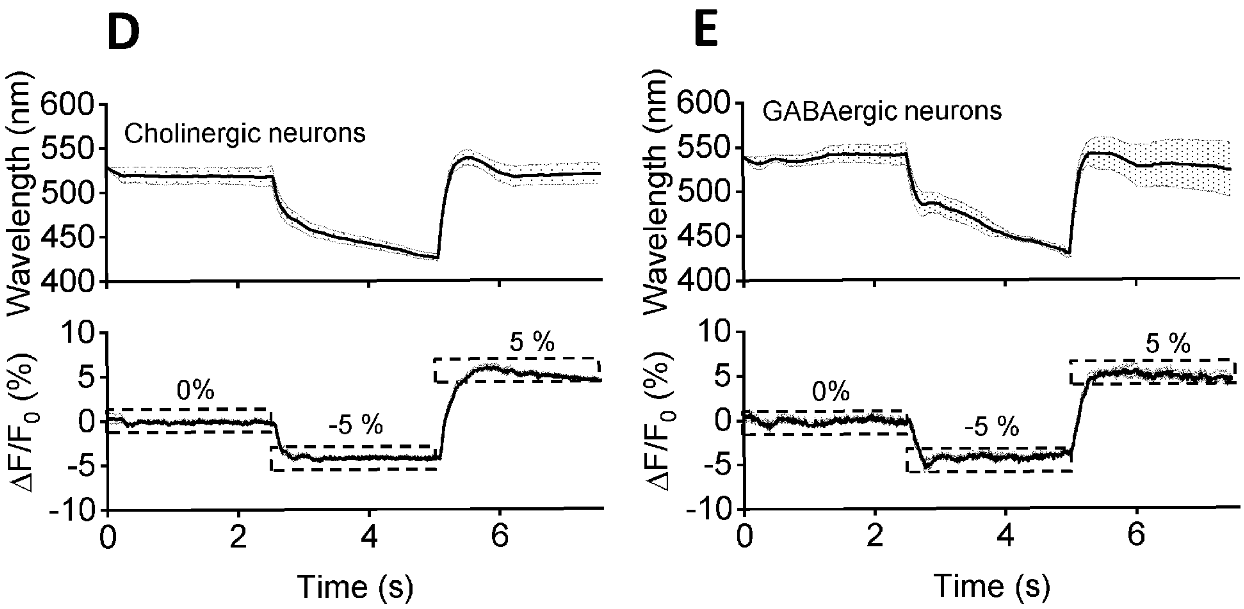

**Figure 5 F and G**

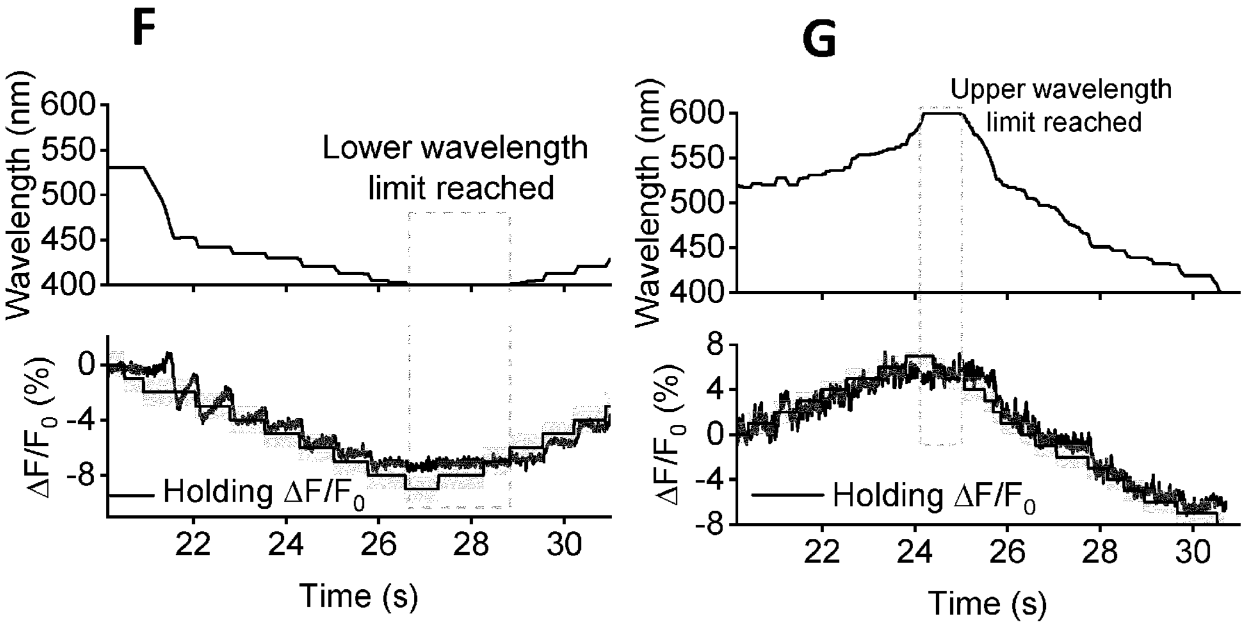

Figure 6 A

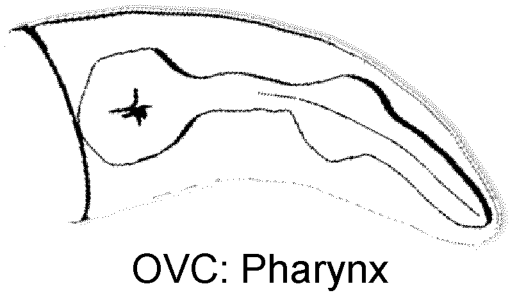

OVC: Pharynx

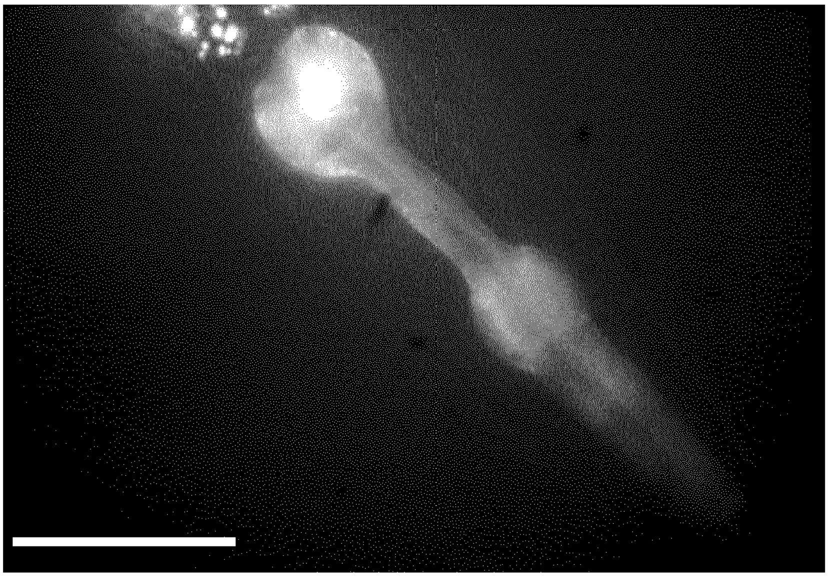

Figure 6 B and C

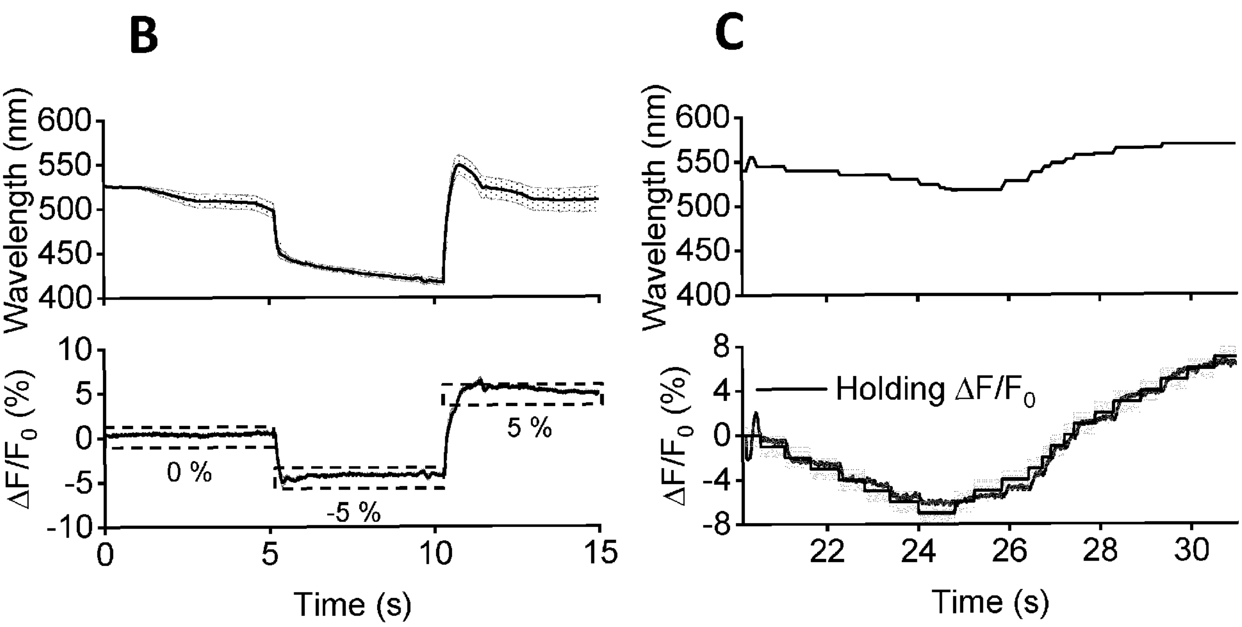

Figure 6 D and E

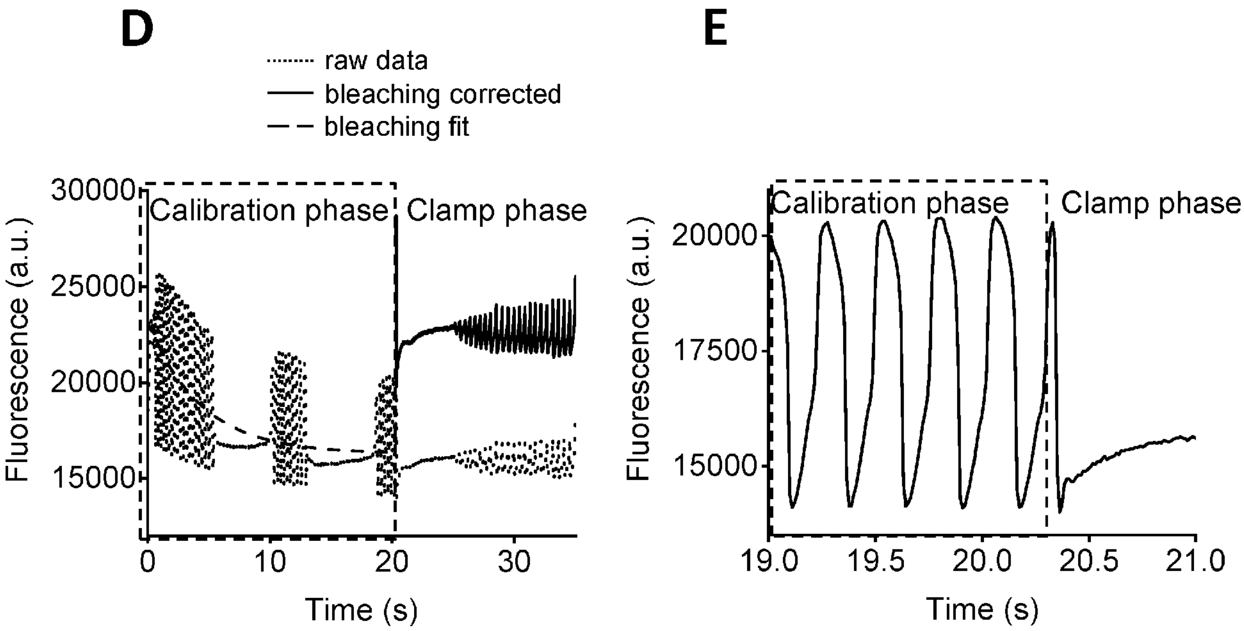

**Figure 6 F and G**

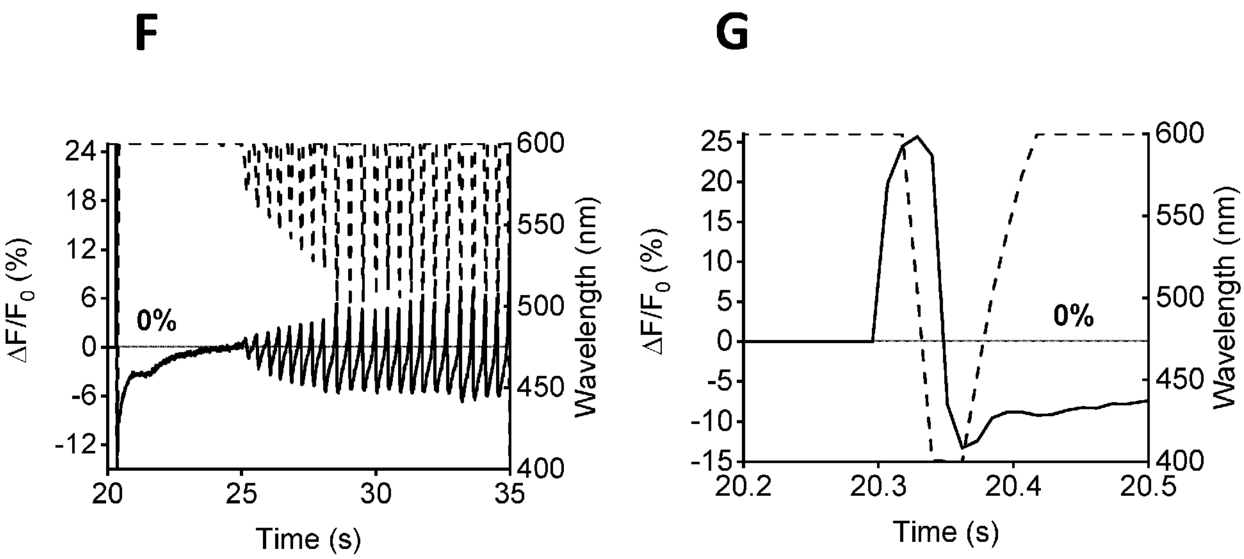

**Figure 6 H and I**

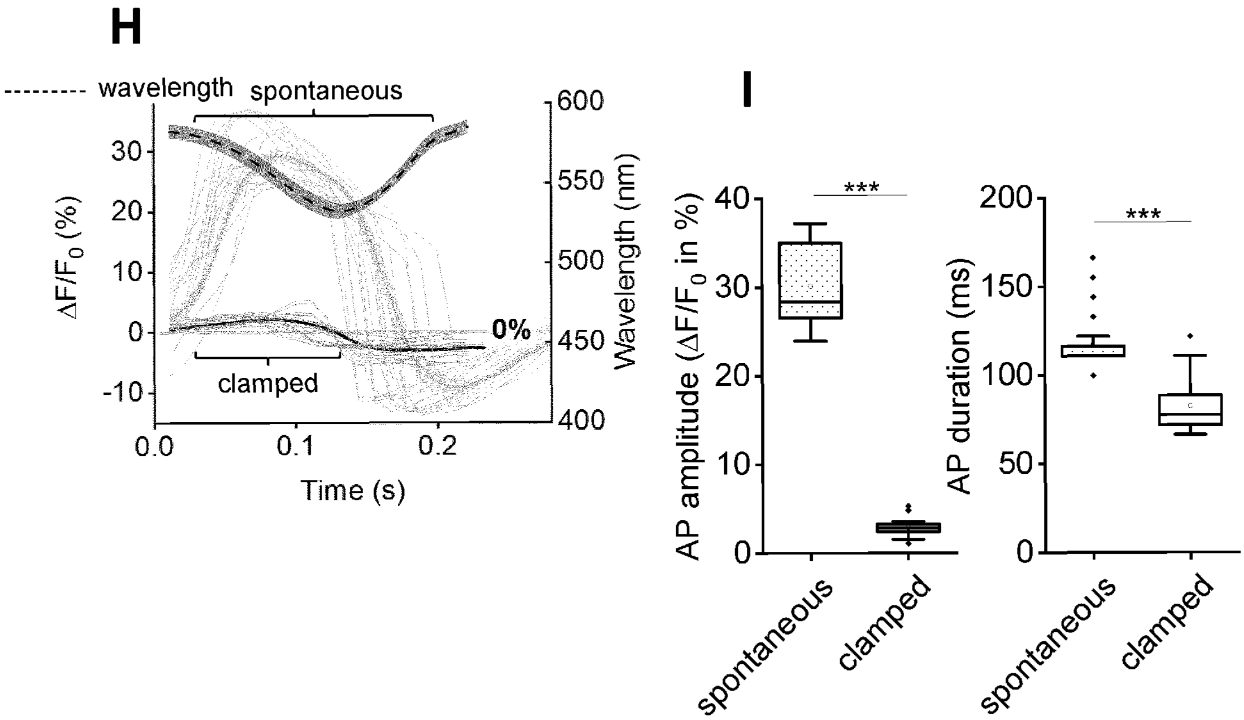

# Figure 7 A

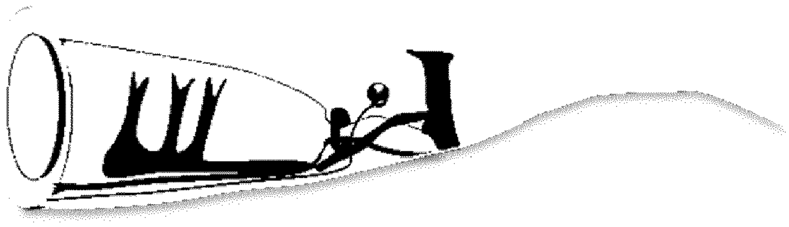

OVC: DVB

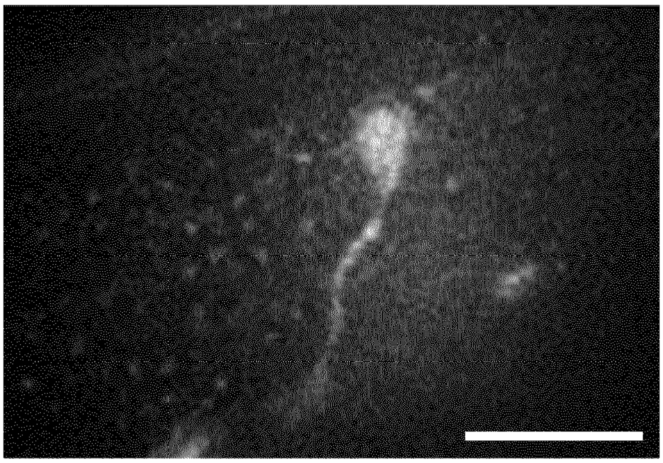

## Figure 7 B

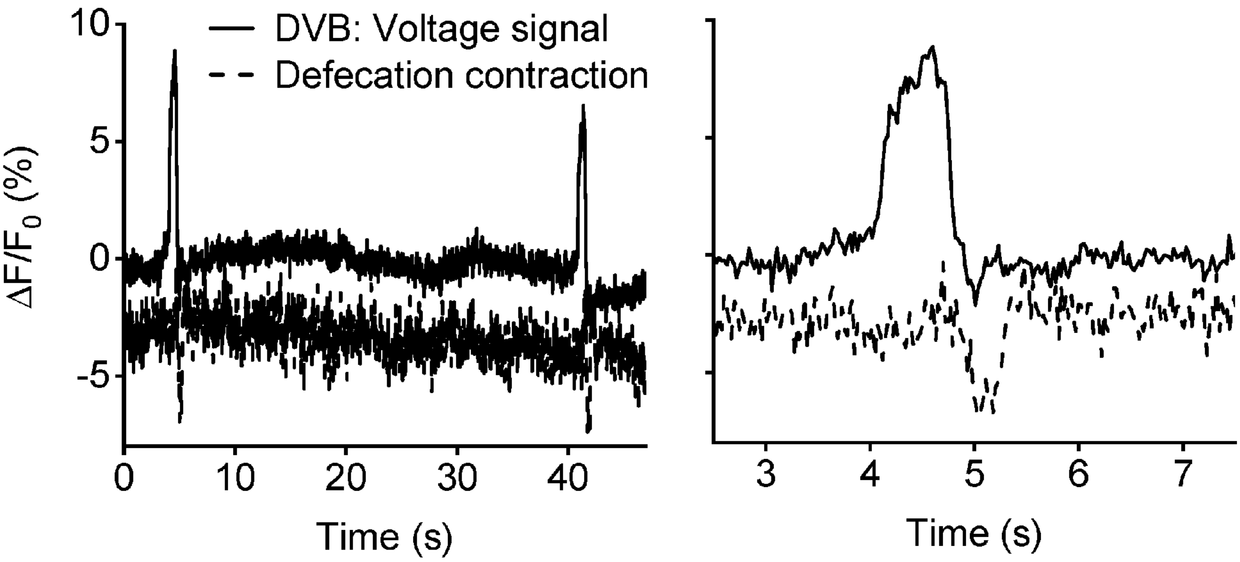

DVB: Voltage signal
Defecation contraction
ΔF/F0 (%)
10
5
0
-5
0
10
20
30
40
Time (s)
3
4
5
6
7
Time (s)

Figure 7 C and D

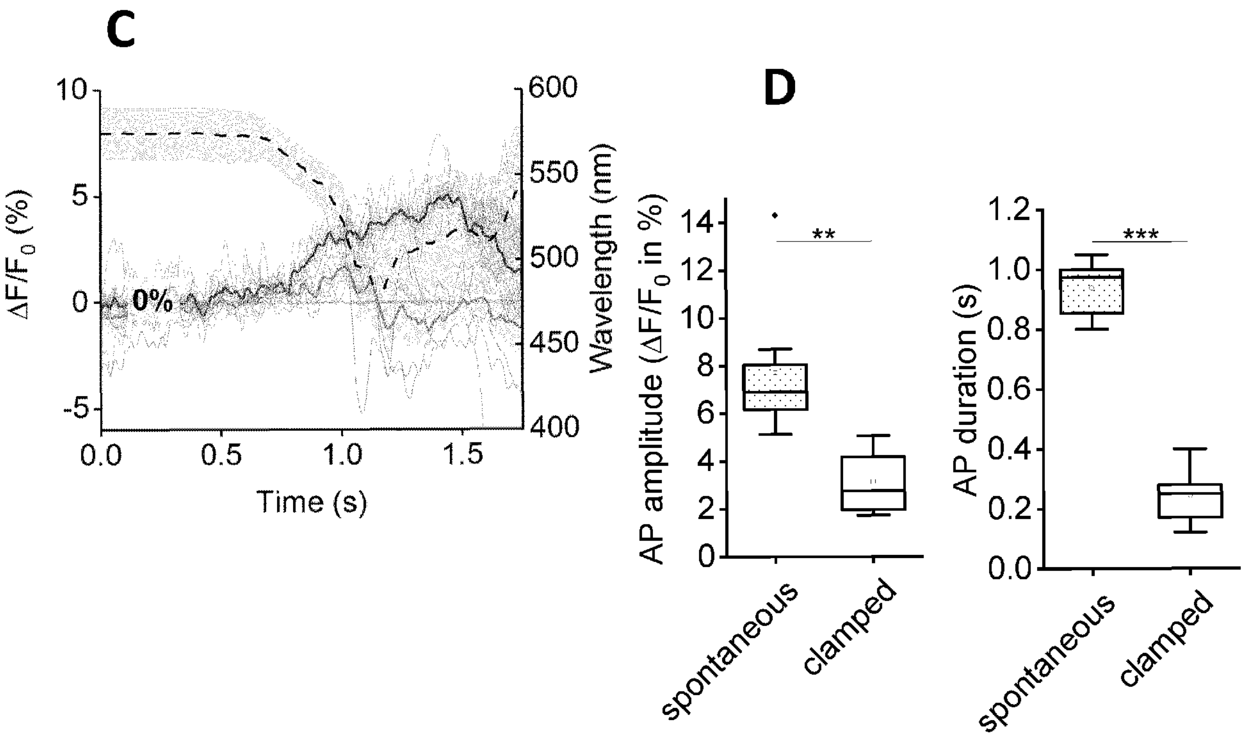

Figure 7 E and F

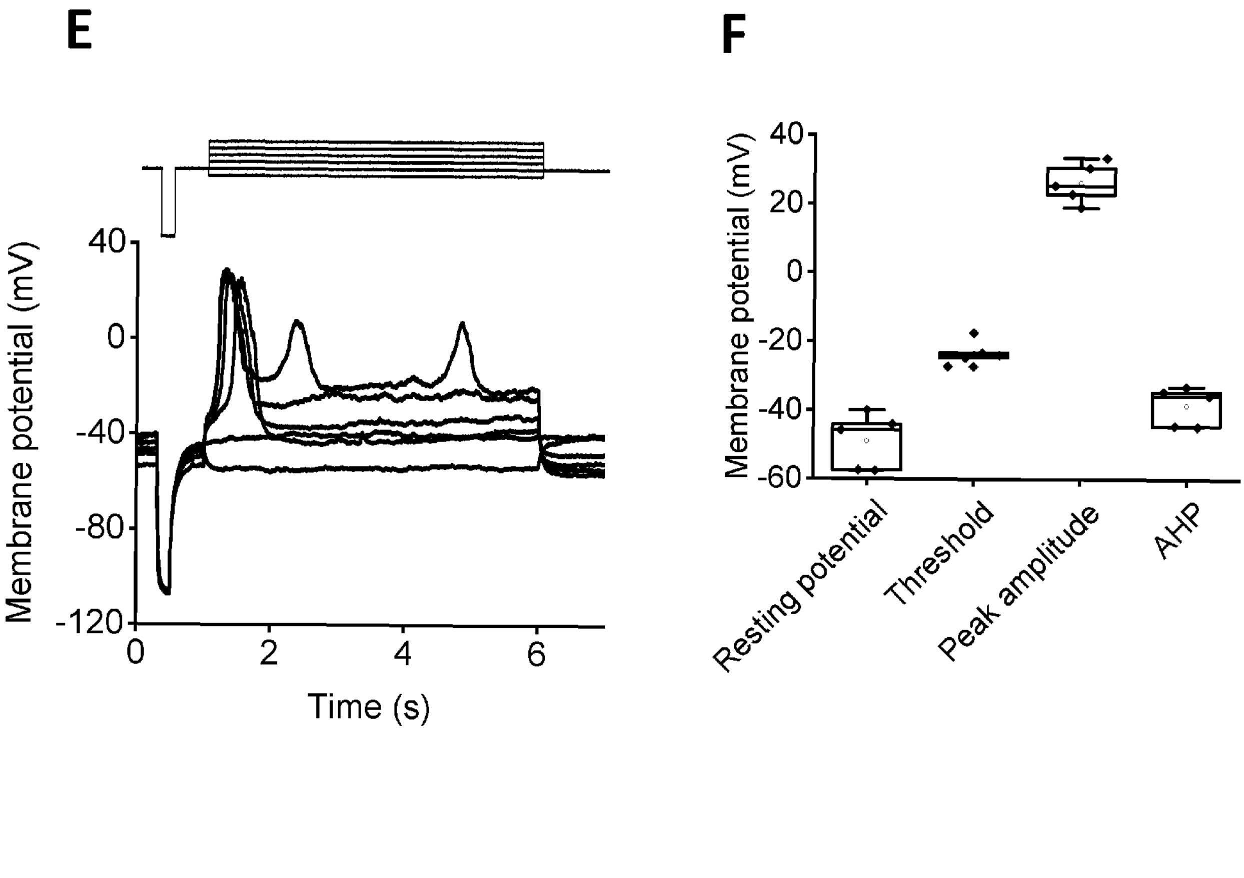

Europäisches Patentamt
European Patent Office
Office européen des brevets

# EUROPEAN SEARCH REPORT

Application Number
EP 21 16 2331

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ENTCHEVA EMILIA ET AL: "Cardiac optogenetics: a decade of enlightenment", NATURE REVIEWS CARDIOLOGY, NATURE PUBLISHING GROUP, GB, vol. 18, no. 5, 18 December 2020 (2020-12-18), pages 349-367, XP037429614, ISSN: 1759-5002, DOI: 10.1038/S41569-020-00478-0 [retrieved on 2020-12-18] | 1-7 | INV. C12N5/077 G01N21/64 G01N33/487 |
| Y | * ig. 3 and caption * | 8-15 | |
| | ----- | | |
| X | STREIT JONAS ET AL: "Dynamic all-optical drug screening on cardiac voltage-gated ion channels", SCIENTIFIC REPORTS, vol. 8, no. 1, 18 January 2018 (2018-01-18), XP55845587, DOI: 10.1038/s41598-018-19412-z Retrieved from the Internet: URL:http://www.nature.com/articles/s41598-018-19412-z.pdf> | 1-7 | |
| Y | * Fig. 2; section "Methods"; p.3-4 * | 8-15 | TECHNICAL FIELDS SEARCHED (IPC) C12N G01N |
| | ----- | | |
| X | WO 2020/072427 A1 (UNIV CORNELL [US]; UNIV GEORGE WASHINGTON [US]) 9 April 2020 (2020-04-09) | 1-8 | |
| Y | * abstract; claims 24, 37 * | 9-15 | |
| | ----- | | |

The present search report has been drawn up for all claims

1

| Place of search | Date of completion of the search | Examiner |
|---|---|---|
| Munich | 29 September 2021 | Hohwy, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 16 2331

This annex lists the patent family members relating to the patent documents cited in the above-mentioned European search report. The members are as contained in the European Patent Office EDP file on
The European Patent Office is in no way liable for these particulars which are merely given for the purpose of information.

29-09-2021

| Patent document cited in search report | Publication date | Patent family member(s) | Publication date |
|---|---|---|---|
| WO 2020072427 A1 | 09-04-2020 | EP 3860337 A1 | 11-08-2021 |
| | | WO 2020072427 A1 | 09-04-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **AKERBOOM, J. et al.** Genetically encoded calcium indicators for multi-color neural activity imaging and combination with optogenetics. *Frontiers in molecular neuroscience,* 2013, vol. 6, 2
- **AVERY, L. ; THOMAS, J.H.** Feeding and Defecation. Cold Spring Harbor Laboratory Press, 1997, 679-716
- **BERGS, A. et al.** Rhodopsin optogenetic toolbox v2.0 for light-sensitive excitation and inhibition in Caenorhabditis elegans. *PloS one,* 2018, vol. 13, e0191802
- **BERNDT, A. ; YIZHAR, O. ; GUNAYDIN, L. A. ; HEGEMANN, P. ; DEISSEROTH, K.** Bi-stable neural state switches. *Nature neuroscience,* 2009, vol. 12, 229-234
- **CHEN, T.-W. et al.** Ultrasensitive fluorescent proteins for imaging neuronal activity. *Nature,* 2013, vol. 499, 295-300
- **DAWYDOW, A. et al.** Channelrhodopsin-2-XXL, a powerful optogenetic tool for low-light applications. *Proceedings of the National Academy of Sciences of the United States of America,* 2014, vol. 111, 13972-13977
- **EDELSTEIN, A. ; AMODAJ, N. ; HOOVER, K. ; VALE, R. ; STUURMAN, N.** Computer control of microscopes using µManager. *Current protocols in molecular biology,* 2010
- **EVANS, T.** Transformation and microinjection. *WormBook; 10.1895/wormbook.1.108.1,* 2006
- **FAN, L. Z. et al.** All-Optical Electrophysiology Reveals the Role of Lateral Inhibition in Sensory Processing in Cortical Layer 1. *Cell,* 2020, vol. 180, 521-535
- **FRANCIS, M. M. ; MELLEM, J. E. ; MARICQ, A. V.** Bridging the gap between genes and behavior: recent advances in the electrophysiological analysis of neural function in Caenorhabditis elegans. *Trends in Neurosciences,* 2003, vol. 26, 90-99
- **GONG, Y. ; LI, J. Z. ; SCHNITZER, M. J.** Enhanced Archaerhodopsin Fluorescent Protein Voltage Indicators. *PloS one,* 2013, vol. 8, e66959
- **GONG, Y. ; WAGNER, M. J. ; ZHONG LI, J. ; SCHNITZER, M. J.** Imaging neural spiking in brain tissue using FRET-opsin protein voltage sensors. *Nature communications,* 2014, vol. 5, 3674
- **GOVORUNOVA, E. G. ; SINESHCHEKOV, O. A. ; JANZ, R. ; LIU, X. ; SPUDICH, J. L.** NEUROSCIENCE. Natural light-gated anion channels: A family of microbial rhodopsins for advanced optogenetics. *Science,* 2015, vol. 349, 647-650
- **AZIMI HASHEMI, N. et al.** Rhodopsin-based voltage imaging tools for use in muscles and neurons of Caenorhabditis elegans. *Proceedings of the National Academy of Sciences of the United States of America,* 2019, vol. 116, 17051-17060
- **HOCHBAUM, D. R. et al.** All-optical electrophysiology in mammalian neurons using engineered microbial rhodopsins. *Nature methods,* 2014, vol. 11, 825-833
- **HUSSON, S. J. et al.** Microbial light-activatable proton pumps as neuronal inhibitors to functionally dissect neuronal networks in C. elegans. *PloS one,* 2012, vol. 7, e40937
- **KRALJ, J. M. ; DOUGLASS, A. D. ; HOCHBAUM, D. R. ; MACLAURIN, D. ; COHEN, A. E.** Optical recording of action potentials in mammalian neurons using a microbial rhodopsin. *Nature methods,* 2011, vol. 9, 90-95
- **LIEWALD, J. F. et al.** Optogenetic analysis of synaptic function. *Nature methods,* 2008, vol. 5, 895-902
- **LIN, M. Z. ; SCHNITZER, M. J.** Genetically encoded indicators of neuronal activity. *Nature neuroscience,* 2016, vol. 19, 1142-1153
- **MCINTIRE, S. L. ; JORGENSEN, E. ; KAPLAN, J. ; HORVITZ, H. R.** The GABAergic nervous system of Caenorhabditis elegans. *Nature,* 1993, vol. 364, 337-341
- **MIURA, K. ; RUEDEN, C. ; HINER, M. ; SCHINDELIN, J. ; RIETDORF, J.** *Imagej Plugin Correctbleach V2.0.2,* 2014
- **NEWMAN, J. P et al.** Optogenetic feedback control of neural activity. *eLife,* 2015, vol. 4, e07192
- **PIATKEVICH, K. D. et al.** A robotic multidimensional directed evolution approach applied to fluorescent voltage reporters. *Nature chemical biology,* 2018, vol. 14, 352-360
- **RICHMOND, J. E. ; DAVIS, W. S. ; JORGENSEN, E. M.** UNC-13 is required for synaptic vesicle fusion in C. elegans. *Nature neuroscience,* 1999, vol. 2, 959-964

- **SCHULTHEIS, C. ; LIEWALD, J. F. ; BAMBERG, E. ; NAGEL, G. ; GOTTSCHALK, A.** Optogenetic long-term manipulation of behavior and animal development. *PloS one,* 2011, vol. 6, e18766
- **VIEROCK, J. et al.** BiPOLES: a tool for bidirectional dual-color optogenetic control of neurons. *bioRxiv,* 15 July 2020
- **WANG, H. ; SIEBURTH, D.** PKA controls calcium influx into motor neurons during a rhythmic behavior. *PLoS genetics,* 2013, vol. 9, e1003831
- **ZHANG F ; WANG LP ; BRAUNER M ; LIEWALD JF ; KAY K ; WATZKE N ; WOOD PG ; BAMBERG E ; NAGEL G ; GOTTSCHALK A.** Multimodal fast optical interrogation of neural circuitry. *Nature,* 05 April 2007, vol. 446 (7136), 633-9